(19) Europäisches Patentamt
European Patent Office
Office européen des brevets

(11) EP 0 883 618 B1

(12) EUROPÄISCHE PATENTSCHRIFT

(45) Veröffentlichungstag und Bekanntmachung des Hinweises auf die Patenterteilung:
**16.05.2001 Patentblatt 2001/20**

(21) Anmeldenummer: **96944617.8**

(22) Anmeldetag: **20.12.1996**

(51) Int Cl.[7]: **C07D 405/04**, C09K 19/34, C09K 19/58, C07D 405/14

(86) Internationale Anmeldenummer:
**PCT/EP96/05774**

(87) Internationale Veröffentlichungsnummer:
**WO 97/24351 (10.07.1997 Gazette 1997/30)**

(54) **DIFLUORPHENYLPYRIMIDYLPYRIDIN-DERIVATE UND IHRE VERWENDUNG IN FLÜSSIGKRISTALLINEN MISCHUNGEN**

DIFLUOROPHENYL PYRIMIDYL PYRIDINE DERIVATIVES AND THE USE THEREOF IN LIQUID CRYSTAL MIXTURES

DERIVES DE DIFLUOROPHENYLPYRIMIDYLPYRIDINE ET LEUR UTILISATION DANS DES MELANGES A CRISTAUX LIQUIDES

(84) Benannte Vertragsstaaten:
**DE FR GB**

(30) Priorität: **28.12.1995 JP 34328895**
**21.11.1996 DE 19648171**

(43) Veröffentlichungstag der Anmeldung:
**16.12.1998 Patentblatt 1998/51**

(73) Patentinhaber: **Aventis Research & Technologies GmbH & Co KG**
**65926 Frankfurt am Main (DE)**

(72) Erfinder:
- **NONAKA, Toshiaki**
  **Saitama (JP)**
- **LI, Ji**
  **Tokyo (JP)**
- **TAKEICHI, Ayako**
  **Saitama (JP)**
- **HORNUNG, Barbara**
  **D-63594 Hasselroth (DE)**
- **MANERO, Javier**
  **D-65931 Frankfurt am Main (DE)**
- **SCHMIDT, Wolfgang**
  **D-51143 Köln (DE)**
- **WINGEN, Rainer**
  **D-65795 Hattersheim (DE)**

(74) Vertreter: **Isenbruck, Günter, Dr. et al**
**Patent- und Rechtsanwälte,**
**Bardehle-Pagenberg-Dost-Altenburg-Geissler-Isenbruck**
**Theodor-Heuss-Anlage 12**
**68165 Mannheim (DE)**

(56) Entgegenhaltungen:
**EP-A- 0 332 025**
**WO-A-92/12974**
**DE-A- 4 030 603**
**US-A- 4 668 425**

EP 0 883 618 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Neben nematischen und cholesterischen Flüssigkristallen werden in jüngerer Zeit auch optisch aktive geneigt smektische (ferroelektrische) Flüssigkristalle in kommerziellen Displayvorrichtungen verwendet.

Clark und Lagerwall konnten zeigen, daß der Einsatz ferroelektrischer Flüssigkristalle (FLC) in sehr dünnen Zellen zu optoelektrischen Schalt- oder Anzeigeelementen führt, die im Vergleich zu den herkömmlichen TN ("twisted nematic")-Zellen um bis zu einem Faktor 1000 schnellere Schaltzeiten haben (siehe z. B. EP-A 0 032 362). Aufgrund dieser und anderer günstiger Eigenschaften, z. B. der bistabilen Schaltmöglichkeit und des nahezu blickwinkelunabhängigen Kontrasts, sind FLCs grundsätzlich für Anwendungsgebiete wie Computerdisplays gut geeignet.

Für die Verwendung von FLCs in elektrooptischen oder vollständig optischen Bauelementen benötigt man entweder Verbindungen, die geneigte bzw. orthogonale smektische Phasen ausbilden und selbst optisch aktiv sind, oder man kann durch Dotierung von Verbindungen, die zwar solche smektischen Phasen ausbilden, selbst aber nicht optisch aktiv sind, mit optisch aktiven Verbindungen ferroelektrische smektische Phasen induzieren. Die gewünschte Phase soll dabei über einen möglichst großen Temperaturbereich stabil sein.

Zur Erzielung eines guten Kontrastverhältnisses in elektrooptischen Bauelementen ist eine einheitliche planare Orientierung der Flüssigkristalle nötig. Eine gute Orientierung in der $S_A$ und $S^*_C$-Phase läßt sich z. B. erreichen, wenn die Phasenfolge der Flüssigkristallmischung mit abnehmender Temperatur lautet:

$$\text{Isotrop} \rightarrow N^* \rightarrow S_A \rightarrow S^*_C$$

Voraussetzung ist, daß der Pitch (Ganghöhe der Helix) in der N*-Phase sehr groß (größer 10 μm) oder, noch besser, völlig kompensiert ist (siehe z. B.: T. Matsumoto et al., Proc. of the 6th Int. Display Research Conf., Japan Display, Sept. 30- Okt. 2, 1986, Tokyo, Japan, S. 468-470; M. Murakami et al., ibid., S. 344-347). Dies erreicht man z. B., indem man zu der chiralen Flüssigkristallmischung, die in der N*-Phase z. B. eine linksdrehende Helix aufweist, einen oder mehrere optisch aktive Dotierstoffe, die eine rechtsdrehende Helix induzieren, in solchen Mengen hinzugibt, daß die Helix kompensiert wird.

Für die Verwendung des SSFLCD-Effektes (Surface Stabilized Ferroelectric Liquid Crystal Display) von Clark und Lagerwall zur einheitlichen, planaren Orientierung ist ferner Voraussetzung, daß der Pitch in der smektischen C*-Phase wesentlich größer ist als die Dicke des Anzeigeelementes (Mol. Cryst. Liq. Cryst. 1983, 94, 213 und 1984, 114, 151. Dies erreicht man, wie im Fall des cholesterischen Pitches, durch Verwendung von Dotierstoffen mit entgegengesetztem Drehsinn der Helix.

Die optische Schaltzeit T [μs] ferroelektrischer Flüssigkristallsysteme, die möglichst kurz sein soll, hängt von der Rotationsviskosität des Systems Y [mPas], der spontanen Polarisation $P_s$ [nC/cm$^2$] und der elektrischen Feldstärke E [V/m] ab nach der Beziehung

$$T \sim \frac{Y}{P_s \cdot E}$$

Da die Feldstärke E durch den Elektrodenabstand im elektrooptischen Bauteil und durch die angelegte Spannung festgelegt ist, muß das ferroelektrische Anzeigemedium niedrigviskos sein und eine hohe spontane Polarisation aufweisen, damit eine kurze Schaltzeit erreicht wird.

Schließlich wird neben thermischer, chemischer und photochemischer Stabilität eine kleine optische Anisotropie $\Delta n$, und eine geringe positive oder vorzugsweise negative dielektrische Anisotropie $\Delta\varepsilon$ verlangt (siehe z. B. S. T. Lagerwall et al., "Ferroelectric Liquid Crystals for Displays" SID Symposium, Oct. Meeting 1985, San Diego, Ca, USA).

Die Gesamtheit dieser Forderungen ist nur mit Mischungen aus mehreren Komponenten zu erfüllen. Als Basis (oder Matrix) dienen dabei bevorzugt Verbindungen, die möglichst selbst bereits die gewünschte Phasenfolge $I \rightarrow N \rightarrow S_A \rightarrow S_C$ aufweisen. Weitere Komponenten der Mischung werden oftmals zur Schmelzpunktserniedrigung und zur Verbreiterung der $S_C$- und meist auch N-Phase, zum Induzieren der optischen Aktivität, zur Pitch-Kompensation und zur Anpassung der optischen und dielektrischen Anisotropie zugesetzt, wobei aber beispielsweise die Rotationsviskosität möglichst nicht vergrößert werden soll.

Ferroelektrische Flüssigkristallanzeigen lassen sich auch durch Nutzung des DHF (Distorted Helix Formation)-Effektes oder des PSFLCD-Effektes (Pitch Stabilized Ferroelectric Liquid Crystal Display, auch SBF = Short Pitch Bistable Ferroelectric Effect genannt) betreiben. Der DHF-Effekt wurde von B. I. Ostrovski in Advances in Liquid Crystal Research and Applications, Oxford/Budapest, 1980, 469 ff. beschrieben, der PSFLCD-Effekt ist in DE-A 39 20 625 bzw. EP-A 0 405 346 beschrieben. Zur Nutzung dieser Effekte wird im Gegensatz zum SSFLCD-Effekt ein flüssigkristallines Material mit einem kurzen $S_C$-Pitch benötigt.

Derivate des 1,2-Difluorbenzols als Flüssigkristalle bzw. als Komponenten flüssigkristalliner Mischungen sind beispielsweise aus DE-A 38 07 871 und DE-A 38 07 862 bekannt.
Pyridylpyrimidine sind beispielsweise aus WO-A 92/12974 und US 4,668,425 bekannt.

Da aber die Entwicklung, insbesondere von ferroelektrischen Flüssigkristallmischungen, noch in keiner Weise als abgeschlossen betrachtet werden kann, sind die Hersteller von Displays an den unterschiedlichsten Komponenten für Mischungen interessiert. Dieses u. a. auch deshalb, weil erst das Zusammenwirken der flüssigkristallinen Mischungen mit den einzelnen Bauteilen der Anzeigevorrichtung bzw. der Zellen (z. B. der Orientierungsschicht) Rückschlüsse auf die Qualität auch der flüssigkristallinen Mischungen zuläßt.

Aufgabe der vorliegenden Erfindung war es daher, Verbindungen bereitzustellen, die in flüssigkristallinen Mischungen geeignet sind, das Eigenschaftsprofil dieser Mischungen zu verbessern.

Es wurde nun überraschend gefunden, daß Difluorphenylpyrimidylpyridin-Derivate der Formel (I) in besonderer Weise zum Einsatz in Flüssigkristallmischungen geeignet sind.

Ein Gegenstand der Erfindung sind daher Verbindungen der Formel (I),

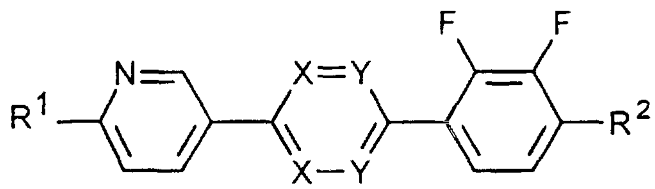 (I)

in der die Symbole folgende Bedeutungen haben:

X ist N und Y ist CH oder X ist CH und Y ist N;
$R^1$, $R^2$ sind gleich oder verschieden

a) eine unverzweigte oder verzweigte Alkylkette mit 1 bis 20 C-Atomen, wobei
aa) eine oder mehrere nicht benachbarte und nicht terminale $CH_2$-Gruppen durch -O-, -CO-O-, -O-CO-, -O-CO-O-, $-Si(CH_3)_2-$ ersetzt sein können, und/oder
ab) ein oder mehrere H-Atome durch F ersetzt sein können, und/oder
ac) die terminale $CH_3$-Gruppe durch eine der folgenden chiralen Gruppen (optisch aktiv oder racemisch) ersetzt sein kann:

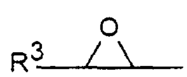 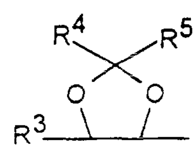

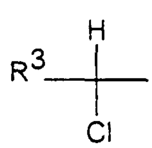 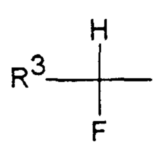 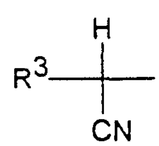

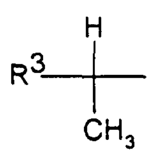 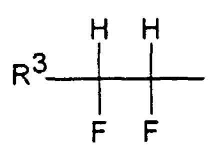

b) Wasserstoff, wobei nur einer der beiden Reste $R^1$, $R^2$ H sein darf,

mit der Maßgabe, daß $R^1$ nicht über -CO-O- oder -O-CO-O- an den Pyridinring gebunden sein darf;
$R^3$, $R^4$, $R^5$ sind gleich oder verschieden Wasserstoff oder ein geradkettiger oder verzweigter Alkylrest mit 1-16 C-Atomen (mit oder ohne asymmetrisches C-Atom), wobei auch eine oder mehrere nicht benachbarte, nicht terminale $CH_2$-Gruppen durch -O- ersetzt sein können, und/oder wobei ein oder mehrere H-Atome des Alkylrestes durch -F substituiert sein können; $R^4$ und $R^5$ können zusammen auch $-(CH_2)_4-$ oder $-(CH_2)_5-$ sein, wenn sie an ein Dioxolan-System gebunden sind.

Bevorzugt sind Verbindungen der Formel (I), bei denen die Symbole folgende Bedeutungen haben.

$R^1$, $R^2$ sind gleich oder verschieden

a) eine unverzweigte oder verzweigte Alkylkette mit 1 bis 16 C-Atomen, wobei
aa) eine oder mehrere nicht benachbarte und nicht terminale $CH_2$-Gruppen durch -O-, -CO-O-, -O-CO-, -O-CO-O-, $-Si(CH_3)_2-$ ersetzt sein können, und/oder
ab) ein oder mehrere H-Atome durch F ersetzt sein können, und/oder
ac) die terminale $CH_3$-Gruppe durch eine der folgenden chiralen Gruppen (optisch aktiv oder racemisch) ersetzt sein kann:

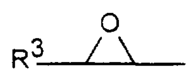

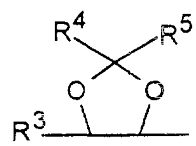

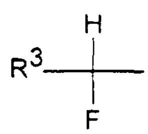

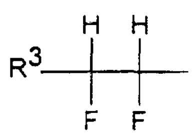

b) Wasserstoff, wobei nur einer der beiden Reste $R^1$, $R^2$ H sein darf,

mit der Maßgabe, daß $R^1$ nicht über -CO-O- oder -O-CO-O- an den Pyridinring gebunden sein darf;

$R^3$, $R^4$, $R^5$ sind gleich oder verschieden Wasserstoff oder ein geradkettiger oder verzweigter Alkylrest mit 1-13 C-Atomen, wobei auch eine oder mehrere nicht benachbarte, nicht terminale $CH_2$-Gruppen durch -O- ersetzt sein können und/oder wobei ein oder mehrere H-Atome des Alkylrestes durch -F substituiert sein können; $R^4$ und $R^5$ können zusammen auch $-(CH_2)_4-$ oder $-(CH_2)_5-$ sein, wenn sie an ein Dioxolan-System gebunden sind.

Besonders bevorzugt sind Verbindungen der Formel (I). bei denen die Symbole folgende Bedeutungen haben:

$R^1$, $R^2$ sind gleich oder verschieden

a) eine unverzweigte oder verzweigte Alkylkette mit 1 bis 12 C-Atomen, wobei
aa) eine oder mehrere nicht benachbarte und nicht terminale $CH_2$-Gruppen durch -O-, -CO-O-, -O-CO-, -O-CO-O- ersetzt sein können, und/oder
ab) ein oder mehrere H-Atome durch F ersetzt sein können, und/oder
ac) die terminale $CH_3$-Gruppe durch eine der folgenden chiralen Gruppen (optisch aktiv oder racemisch) ersetzt sein kann:

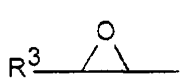

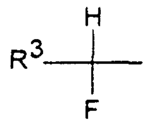

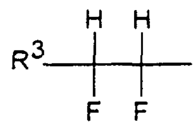

b) Wasserstoff, wobei nur einer der beiden Reste $R^1$, $R^2$ H sein darf,

mit der Maßgabe, daß $R^1$ nicht über -CO-O- oder -O-CO-O- an den Pyridinring gebunden sein darf;

$R^3$ ist gleich oder verschieden Wasserstoff oder ein geradkettiger oder verzweigter Alkylrest mit 1 bis 10 C-Atomen, wobei auch eine oder zwei nicht benachbarte, nicht terminale $CH_2$-Gruppen durch -O- ersetzt sein können.

Ganz besonders bevorzugt sind Verbindungen der Formel (I), bei denen die Symbole folgende Bedeutungen haben:

$R^1$, $R^2$ sind gleich oder verschieden

a) eine unverzweigte oder verzweigte Alkylkette mit 1 bis 12 C-Atomen, wobei
aa) eine oder mehrere nicht benachbarte und nicht terminale $CH_2$-Gruppen durch -O-, -CO-O-, -O-CO-, -O-CO-O- ersetzt sein können, und/oder
ab) die terminale $CH_3$-Gruppe durch eine der folgenden chiralen Gruppen (optisch aktiv oder racemisch) ersetzt sein kann:

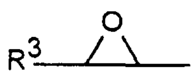

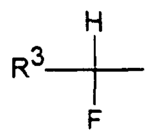

b) Wasserstoff, wobei nur einer der beiden Reste $R^1$, $R^2$ H sein darf,

mit der Maßgabe, daß $R^1$ nicht über -CO-O- oder -O-CO-O- an den Pyridinring gebunden sein darf;

$R^3$ ist gleich oder verschieden Wasserstoff oder ein geradkettiger oder verzweigter Alkylrest mit 1 bis 10 C-Atomen, wobei auch eine oder zwei nicht benachbarte, nicht terminale $CH_2$-Gruppen durch -O- ersetzt sein können.

Die Herstellung der erfindungsgemäßen Verbindungen erfolgt nach an sich literaturbekannten Methoden, wie sie in Standardwerken zur Organischen Synthese, z. B. Houben-Weyl, Methoden der Organischen Chemie, Georg-Thieme-Verlag, Stuttgart, beschrieben werden.
Die Herstellung erfolgt dabei unter Reaktionsbedingungen, die für die genannten Umsetzungen bekannt und geeignet sind. Dabei kann man auch von an sich bekannten, hier nicht näher erwähnten Varianten Gebrauch machen.
Die Ausgangsstoffe können gewünschtenfalls auch in situ gebildet werden, und zwar derart, daß man sie aus dem Reaktionsgemisch nicht isoliert, sondern sofort weiter zu den Verbindungen der Formel (I) umsetzt.
Beispielhaft sind in den Schemata 1 und 2 Synthesewege zu Verbindungen der Formel (I) angegeben, wobei auch andere Verfahren denkbar und möglich sind.
Die erfindungsgemäßen Verbindungen der Formel (I) können beispielsweise nach folgenden Syntheseschemata durch Kombination der dort aufgeführten Bausteine mit Hilfe übergangsmetallmetallkatalysierter Kreuzkupplungsreaktionen (z. B. nach EP-A 0 679 619, EP-A 0 694 530, DE-A 42 36 103) hergestellt werden:

Schema 1:

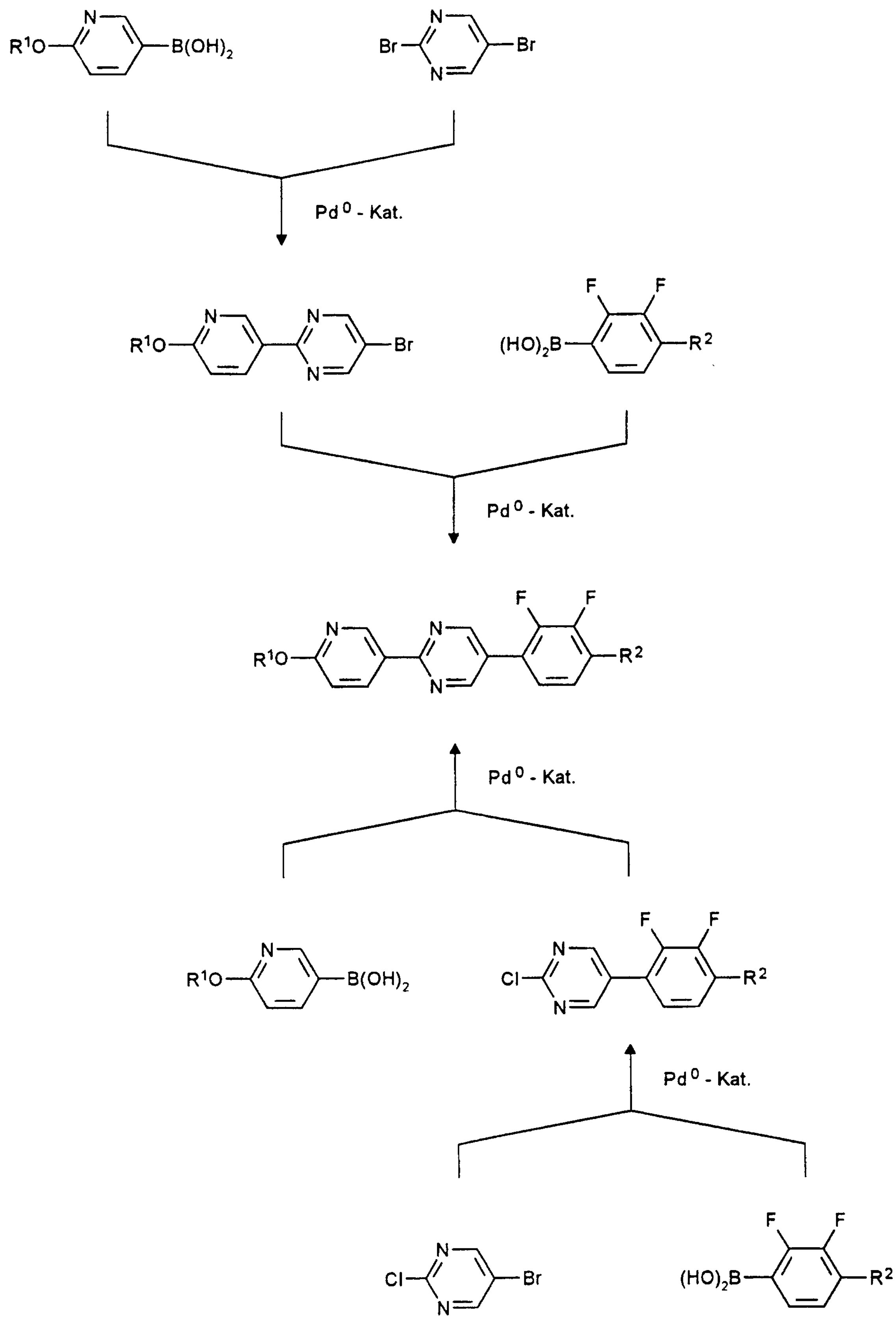

Schema 2:

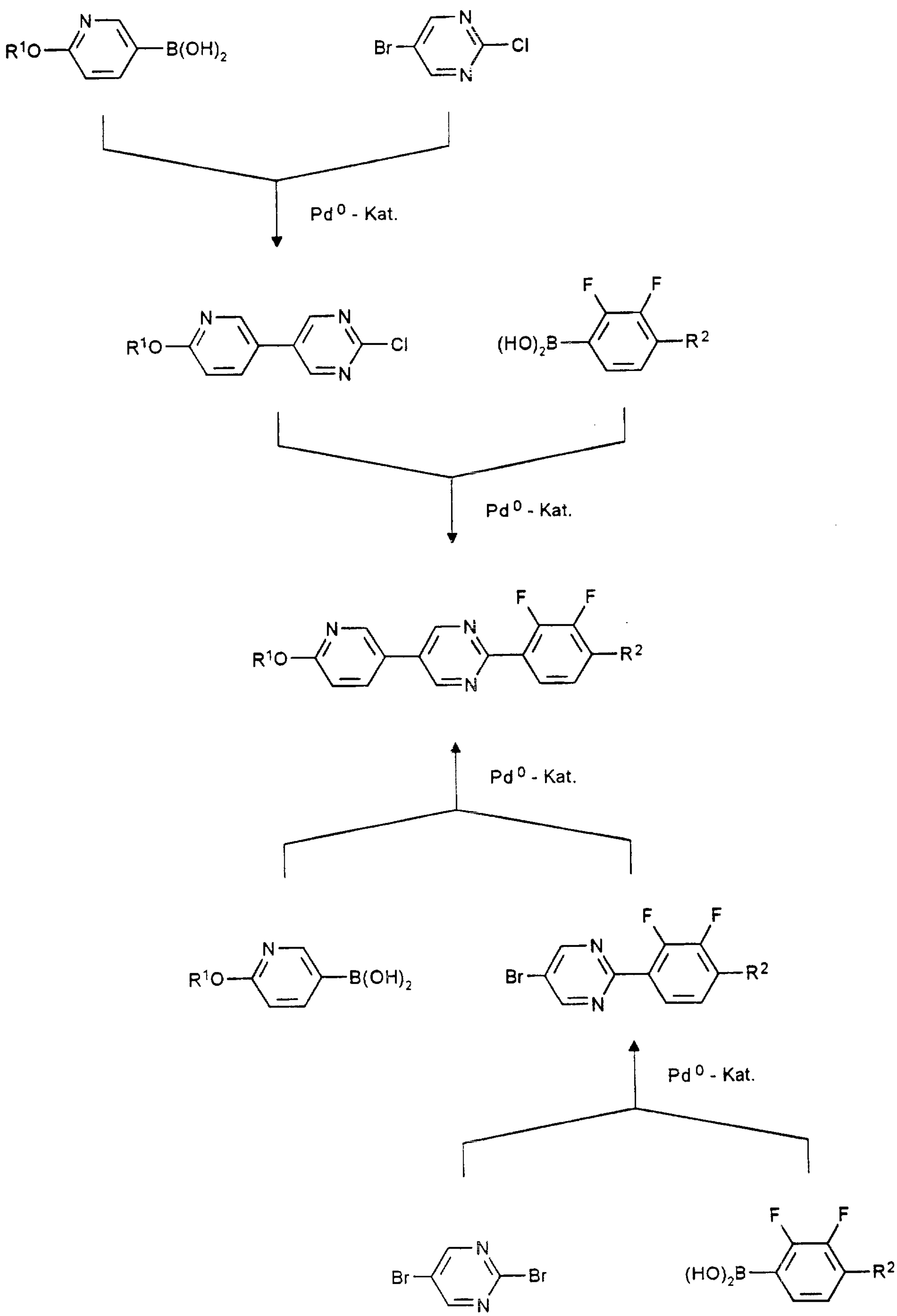

Die Ausgangsverbindungen sind entweder bekannt oder können in Analogie zu bekannten Verbindungen hergestellt werden.

So sind 2,3-Difluorphenylboronsäure und 4-Alkoxy-2,3-difluorphenylboronsäuren beispielsweise in WO 96/00710 beschrieben; auf analoge Weise kann auch 4-Benzyloxy-2,3-difluorphenylboronsäure erhalten werden (WO 96/01246). Beispiele für 4-alkylsubstituierte 2,3-Difluorphenylboronsäuren finden sich in EP-A 0 363 458.

5-Brom-2-chlorpyrimidin läßt sich in zwei Stufen ausgehend von käuflichem 2-Hydroxypyrimidin-hydrochlorid durch Umsetzung mit Brom in wäßriger Lösung (vgl.: D. G. Crosby, R. V. Berthold, J. Org. Chem. 1960, 25, 1916-1919) und anschließender Chlorierung mit $POCl_3$/Triethylamin erhalten (vgl.: D. J. Brown, J. M. Lyall, Australian J. Chem. 1964, 17, 794-802).

Die Synthese von 2,5-Dibrompyrimidin ist beispielhaft in US 5,371,224 beschrieben.

Die Herstellung von 2-Alkoxypyridin-5-boronsäuren kann ausgehend von käuflichem 2,5-Dibrompyridin gemäß nachfolgendem Schema erfolgen:

Schema 3:

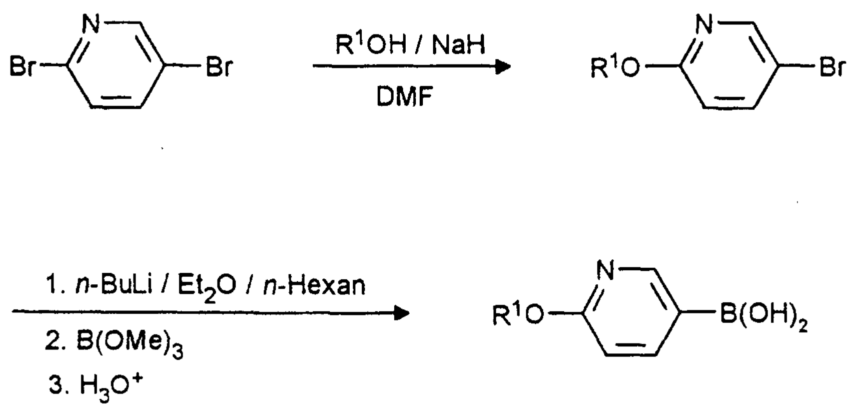


Ether der Formel (I) sind auch durch Veretherung der entsprechenden Phenole ($R^2$ = OH) erhältlich, wobei das Phenol zweckmäßig zunächst in ein entsprechendes Metallderivat, z. B. durch Behandeln mit NaH, $NaNH_2$, NaOH, KOH, $Na_2CO_3$ oder $K_2CO_3$ in das entsprechende Alkalimetallphenolat überführt wird. Dieses kann dann mit dem entsprechenden Alkylhalogenid, Alkylsulfonat oder Dialkylsulfat umgesetzt werden, zweckmäßig in einem inerten Lösungsmittel wie Aceton, 1,2-Dimethoxyethan, DMF (Dimethylformamid) oder Dimethylsulfoxid, oder auch mit einem Überschuß an wäßriger oder wäßrig-alkoholischer NaOH oder KOH bei Temperaturen zwischen etwa 20°C und 100°C.

Geeignet zur Veretherung ist z. B. auch die Umsetzung von Phenolen der Formel (I) mit Alkoholen unter Verwendung von Azodicarbonsäurediethylester und Triphenylphosphin nach der sogenannten Mitsunobu-Methode (vgl.: O. Mitsunobu, Synthesis 1981, 1-28 oder L. Navailles, H. T. Nguyen, P. Barois, Liq. Cryst. 1996, 20, 653-664)

Die entsprechenden Alkohole, Alkylhalogenide, Alkylsulfonate und Dialkylsulfate sind entweder bekannt oder lassen sich in Analogie zu bekannten Verfahren herstellen.

Phenole der Formel (I) ($R^2$ = OH) können auch durch Abspaltung einer geeigneten Schutzgruppe, z. B. aus der entsprechenden Benzyloxyverbindung durch Hydrierung an Pd/C oder aus der entsprechenden unsubstituierten Verbindung ($R^2$ = H) über Verfahren analog zu WO 96/00710 oder C. C. Dong, M. Hird, J. W. Goodby, P. Styring, K. J. Toyne, Ferroelectrics 1996, 180, 245-257, hergestellt werden.

Ester der Formel (I) können auch durch Veresterung der entsprechenden Carbonsäuren (oder ihrer reaktionsfähigen Derivate) mit Phenolen der Formel (I) ($R^2$ = OH) oder ihren reaktionsfähigen Derivaten erhalten werden. Geeignet hierfür ist z. B. die sogenannte DCC-Methode (DCC = Dicyclohexylcarbodiimid; vgl.: B. Neises, W. Steglich, Angew. Chem. 1978, 90, 556-557). Ester der Formel (I) lassen sich auch aus den entsprechenden Carbonsäuresalzen (Herstellung nach DE-C 4304756) durch Umsetzung mit Phenolen nach DE-A 4427198 erhalten.

Die entsprechenden Carbonsäuren sind entweder bekannt oder können nach bekannten Verfahren hergestellt werden.

Als reaktionsfähige Derivate der genannten Carbonsäuren eignen sich insbesondere die Säurehalogenide, vor allem die Chloride und Bromide, ferner die Anhydride, z. B. auch gemischte Anhydride, Azide oder Ester, insbesondere Alkylester mit 1-4 C-Atomen in der Alkylgruppe.

Als reaktionsfähige Derivate der genannten Phenole kommen insbesondere die entsprechenden Metallphenolate, vorzugsweise eines Alkalimetalls, wie Natrium oder Kalium, in Betracht.

Die Veresterung wird vorteilhaft in Gegenwart eines inerten Lösungsmittels durchgeführt. Gut geeignet sind insbesondere Ether, wie Diethylether, Dibutylether, THF, Dioxan oder Anisol, Ketone, wie Aceton, Butanon oder Cyclohexanon, Amide, wie DMF oder Phosphorsäurehexamethyltriamid, Kohlenwasserstoffe, wie Benzol, Toluol oder Xylol, Halogenkohlenwasserstoffe, wie Tetrachlorkohlenstoff, Dichlormethan oder Tetrachlorethylen und Sulfoxide, wie Dimethylsulfoxid oder Sulfolan.

Die Einführung einer Carboxylat-Gruppe in Verbindungen der Formel (I) gelingt beispielsweise in Analogie zu GB-A 1098387 oder A. M. Roe, R. A. Burton, D. R. Reavill, J. Chem. Soc., Chem. Commun. 1965, 22, 582 durch Lithiierung der entsprechenden unsubstituierten Verbindungen der Formel (I) ($R^2$ = H) mit n-Buthyllithium und Umsetzung mit Kohlendioxid. Eine anschließende Veresterung der so erhaltenen Carbonsäuren der Formel (I) ($R^2$ = COOH) oder ihrer reaktionsfähigen Derivate mit entsprechenden Hydroxyverbindungen (oder ihren reaktionsfähigen Derivaten) kann analog zur zuvor beschriebenen Vorgehensweise erfolgen.

Die genannten Hydroxyverbindungen (oder ihre reaktionsfähigen Derivate) sind entweder bekannt oder können in Analogie zu bekannten Verfahren hergestellt werden.

Die Einführung von Alkylsubstituenten ist auch durch die Umsetzung von Perfluoralkylsulfonsäureestern der Formel (I) ($R^2$ = $-O-SO_2-C_nF_{2n+1}$) mit Alkylboranen oder mit terminalen Alkinen (Heck-Reaktion) unter Palladiumkatalyse (vgl.: EP-A 0 709 357) möglich. Im zweiten Fall wird anschließend zusätzlich katalytisch hydriert. Geeignete Alkylborane lassen sich nach bekannten Methoden, z. B. durch Umsetzung von endständigen Alkenen mit 9-BBN, erhalten; die genannten Perfluoralkylsulfonsäureester sind aus den jeweiligen Phenolen ($R^2$ = OH) durch Veresterung mit den entsprechenden Sulfonsäureanhydriden zugänglich.

Was die Synthese spezieller Reste $R^1$ und $R^2$ angeht, sei zusätzlich beispielsweise verwiesen auf:

EP-B 0 355 008 für Verbindungen mit siliciumhaltigen Seitenketten,
EP-B 0 292 954 für optisch aktive Verbindungen mit Oxiranestereinheit,
EP-B 0 263 437 für optisch aktive Verbindungen mit Oxiranethereinheit,
EP-B 0 361 272 für optisch aktive Verbindungen mit Dioxolanestereinheit,
EP-B 0 351 746 für optisch aktive Verbindungen mit Dioxolanethereinheit,
US 5,051,506 für optisch aktive Verbindungen mit 2,3-Difluoralkyloxy-Einheit,
US 4,798,680 für optisch aktive Verbindungen mit 2-Fluoralkyloxy-Einheit,
US 4,855,429 für optisch aktive Verbindungen mit $\alpha$-Chlorcarbonsäure-Einheit.

Mit der Bereitstellung von Verbindungen der Formel (I) wird ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

In diesem Zusammenhang besitzen die Verbindungen der Formel (I) einen breiten Anwendungsbereich. In Abhängigkeit von der Auswahl der Substituenten können sie als Basismaterialien dienen, aus denen flüssigkristalline Phasen zum überwiegenden Teil zusammengesetzt sind; es können aber auch Verbindungen der Formel (I) flüssigkristallinen Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren.

Gegenstand der Erfindung ist auch die Verwendung von Verbindungen der Formel
(I) in Flüssigkristallmischungen, vorzugsweise ferroelektrischen, insbesondere ferroelektrischen, die in Anzeigevorrichtungen eingesetzt werden, die auf die Nutzung des SSFLCD-Effektes (Surface Stabilized Ferroelectric Liquid Crystal Display) und dabei insbesondere des sogenannten Inverse - oder $_{\mathbf{T}}V_{(min)}$-Modes beruhen.

Weiterhin Gegenstand der Erfindung sind Flüssigkristallmischungen, vorzugsweise ferroelektrische, insbesondere ferroelektrische, die unter Nutzung des SSFLCD-Effektes und dabei insbesondere im sogenannten Inverse- oder $_{\mathbf{T}}V_{(min)}$-Mode betrieben werden, enthaltend eine oder mehrere Verbindungen der Formel (I).

Die erfindungsgemäßen Flüssigkristallmischungen enthalten im allgemeinen 2 bis 35, vorzugsweise 2 bis 25, besonders bevorzugt 2 bis 20 Komponenten.

Sie enthalten im allgemeinen 0,01 bis 80 Gew.-%, vorzugsweise 0,1 bis 60 Gew.-%, besonders bevorzugt 0,1 bis 30 Gew.-%, an einer oder mehreren, vorzugsweise 1 bis 10, besonders bevorzugt 1 bis 5, ganz besonders bevorzugt 1 bis 3, der erfindungsgemäßen Verbindungen der Formel (I).

Weitere Komponenten von Flüssigkristallmischungen, die erfindungsgemäße Verbindungen der Formel (I) enthalten, werden vorzugsweise ausgewählt aus den bekannten Verbindungen mit smektischen und/oder nematischen und/oder cholesterischen Phasen. Dazu gehören z. B.:

- Derivate des Phenylpyrimidins, wie beispielsweise in WO 86/06401, US- 4 874 542 beschrieben,
- metasubstituierte Sechsringaromaten, wie beispielsweise in EP-A 0 578 054 beschrieben,
- Siliciumverbindungen, wie beispielsweise in EP-B 0 355 008 beschrieben,
- mesogene Verbindungen mit nur einer Seitenkette, wie beispielsweise in EP-A 0 541 081 beschrieben,
- Hydrochinonderivate, wie beispielsweise in EP-A 0 603 786 beschrieben,
- Phenylbenzoate, wie beispielsweise bei P. Keller, *Ferroelectrics* 1984, *58*, 3 und J. W. Goodby et al., *Liquid Crystals and Ordered Fluids,* Bd. 4, New York, 1984 beschrieben und
- Thiadiazole, wie beispielsweise in EP-B 0 309 514 beschrieben.

Als chirale, nicht racemische Dotierstoffe kommen beispielsweise in Frage:

- optisch aktive Phenylbenzoate, wie beispielsweise bei P. Keller, Ferroelectrics 1984, 58, 3 und J. W. Goodby et al., Liquid Crystals and Ordered Fluids, Bd. 4, New York, 1984 beschrieben,
- optisch aktive Oxiranether, wie beispielsweise in EP-B 0 263 437 und WO-A 93/13093 beschrieben,
- optisch aktive Oxiranester, wie beispielsweise in EP-B 0 292 954 beschrieben,
- optisch aktive Dioxolanether, wie beispielsweise in EP-B 0 351 746 beschrieben,
- optisch aktive Dioxolanester, wie beispielsweise in EP-B 0 361 272 beschrieben,
- optisch aktive Tetrahydrofuran-2-carbonsäureester, wie beispielsweise in EP-B 0 355 561 beschrieben, und
- optisch aktive 2-Fluoralkylether, wie beispielsweise in EP-B 0 237 007 und US 5,051,506 beschrieben.

Geeignete weitere Mischungskomponenten sind insbesondere in der internationalen Patentanmeldung PCT/EP 96/03154 aufgeführt, auf die hiermit ausdrücklich Bezug genommen wird.

Die Mischungen wiederum können Anwendung finden in elektrooptischen oder vollständig optischen Elementen, z. B. Anzeigeelementen, Schaltelementen, Lichtmodulatoren, Elementen zur Bildbearbeitung und/oder Signalverarbeitung oder allgemein im Bereich der nichtlinearen Optik.

Ferner sind die Mischungen für Feldbehandlung, d. h. zum Betrieb in der Quasi-Bookshelf-Geometrie (QBG) (siehe z. B. H. Rieger et al., SID 91 Digest (Anaheim) 1991, 396) geeignet.

Die erfindungsgemäßen ferroelektrischen Flüssigkristallmischungen eignen sich insbesondere zum Betrieb im sogenannten Inverse- oder $_{\tau}V_{(min)}$-Mode (siehe z. B.: J. C. Jones, M. J. Towler, J. R. Hughes, Displays 1993, 14, Nr. 2, 86-93; M. Koden, Ferroelectrics 1996, 179, 121-129).

Erfindungsgemäße FLC-Mischungen zeichnen sich beim Betrieb im Inverse-Mode durch eine vorteilhafte Leistungskennziffer ("figure of merit") aus, wie sie bei A. J. Slaney, V. Minter, J. C. Jones, Ferroelectrics 1996, 178, 65-74 definiert ist. Sie sind deshalb besonders nützlich für die praktische Anwendung in Schalt- und/oder Anzeigevorrichtungen (Displays).

Flüssigkristalline Mischungen, die Verbindungen der allgemeinen Formel (I) enthalten, sind besonders für die Verwendung in elektrooptischen Schalt- und Anzeigevorrichtungen (Displays) geeignet. Diese Displays sind üblicherweise so aufgebaut, daß eine Flüssigkristallschicht beiderseitig von Schichten eingeschlossen ist, die üblicherweise, in dieser Reihenfolge ausgehend von der LC-Schicht, mindestens eine Orientierungsschicht, Elektroden und eine Begrenzungsscheibe (z. B. aus Glas) sind. Darüberhinaus können sie Abstandshalter, Kleberahmen, Polarisatoren sowie für Farbdisplays dünne Farbfilterschichten enthalten. Weitere mögliche Komponenten sind Antireflex-, Passivierungs-, Ausgleichs- und Sperrschichten sowie elektrisch-nichtlineare Elemente, wie Dünnschichttransistoren (TFT) und Metall-Isolator-Metall-(MIM)-Elemente. Im Detail ist der Aufbau von Flüssigkristalldisplays bereits in einschlägigen Monographien beschrieben (siehe z. B. E. Kaneko, "Liquid Crystal TV Displays: Principles and Applications of Liquid Crystal Displays", KTK Scientific Publishers, 1987).

Ein weiterer Gegenstand der Erfindung ist daher eine Schalt- und/oder Anzeigevorrichtung, insbesondere eine ferroelektrische, enthaltend eine Flüssigkristallmischung, die eine oder mehrere Verbindungen der Formel (I) enthält.

Vorzugsweise wird die erfindungsgemäße Schalt- undloder Anzeigevorrichtung im Normal- oder Inverse-Mode betrieben.

Durch Multiplexansteuerung betriebene ferroelektrische Schalt- und/oder Anzeigevorrichtungen können unter anderem auf zwei unterschiedliche Weisen betrieben werden, die sogenannte normale (Normal-Mode) oder die sogenannte inverse (Inverse Mode auch $_{\tau}V_{(min)}$-Mode). Der Unterschied zwischen beiden liegt im Ansteuerschema und in den verschiedenen Anforderungen an den dielektrischen Tensor des FLC-Materials, d. h. der FLC-Mischung. Einen Überblick geben z. B. J. C. Jones et al. in Displays 1993, 14, Nr. 2, 86-93 im folgenden als "Jones' bezeichnet, und M. Koden in Ferroelectrics 1996, 179, 121-129, sowie die dort aufgeführte Literatur.

Die Schaltcharakteristika einer FLC-Vorrichtung können im allgemeinen durch ein Diagramm dargestellt werden, bei dem die Treiberspannung (V) horizontal und die Breite der Ansteuerpulse (T, Zeit) vertikal aufgetragen sind (siehe z. B. Jones, Abb. 4, 8, 10 und 11).

Eine Schaltkurve wird experimentell bestimmt und teilt die V, T-Fläche in einen schaltenden- und einen nicht schaltenden Bereich. Üblicherweise verkürzt sich bei Erhöhung der Spannung die Pulsbreite. Dieses Verhalten kennzeichnet den sogenannten Normal-Mode (siehe z. B. Jones, Abb. 4).

Bei geeigneten Materialien weist die VT-Kurve jedoch ein Minimum auf (bei der Spannung $V_{(min)}$), wie z. B. bei Jones in den Abbildungen 8, 10 und 11 zu sehen. Dieses Minimum kommt durch Überlagerung von dielektrischer und ferroelektrischer Verdrillung zustande. FLC-Vorrichtungen werden im Inverse-Mode betrieben, wenn die Summe der Zeilen- und Spalten-Treiberspannung im Arbeitstemperaturbereich höher als das Minimum auf der VT-Kurve sind, d. h. $V_{(Zeile)} + V_{(Zeile)} > V_{(min)}$.

Die Erfindung wird durch die nachfolgenden Beispiele weiter erläutert, ohne sie dadurch beschränken zu wollen.

Folgende Abkürzungen werden verwendet:

| | |
|---|---|
| abs. | absolutiert |
| DCC | N,N'-Diyclohexylcarbodiimid |
| DMAP | 4-(Dimethylamino)-pyridin |
| DMF | N, N-Dimethylformamid |
| ges. | gesättigt |
| Klp. | Klärpunkt |
| Schmp. | Schmelzpunkt |
| THF | Tetrahydrofuran |
| V/V | Volumenverhältnis |

Ferner bedeuten: X = kristalliner Zustand, S = smektische Phase (der Index kennzeichnet den Phasentyp), N = nematische Phase, I = isotrope Phase. Die Zahlenangaben zwischen diesen Symbolen geben die Übergangstemperaturen an. Phasenübergangstemperaturen wurden durch DTA, Phasentypen durch optische Polarisationsmikroskopie bestimmt. Alle Temperaturen sind in Grad Celsius angegeben.

Vorstufe 1:

Synthese von 5-Brom-2-hexyloxypyridin

Zu einer Suspension von 300 mmol Natriumhydrid (80 proz. in Mineralöl) in 100 ml trockenem DMF wird bei 50°C unter Schutzgas eine Lösung von 300 mmol 1-Hexanol in 100 ml DMF getropft. Man rührt 1 h bei 80°C und tropft anschließend eine Lösung von 200 mmol 2,5-Dibrompyridin in 150 ml warmen DMF langsam zu. Die Mischung wird anschließend 3-4 h bei 80°C gerührt. Zur Hydrolyse gibt man die abgekühlte Reaktionsmischung auf 1 l Eis/Wasser, extrahiert mehrfach mit Dichlormethan, wäscht die vereinigten org. Extrakte mit ges. Natriumchloridlösung und trocknet sie mit Natriumsulfat. Nach dem Entfernen des Lösemittels i. Vak. wird der Rückstand an Kieselgel 60 mit Dichlormethan als Eluent chromatographiert.
Man erhält 52 g (quant. Ausbeute) einer viskosen Flüssigkeit.

Auf analoge Weise wurden dargestellt:

5-Brom-2-butyloxypyridin
5-Brom-2-octyloxypyridin
5-Brom-2-dodecyloxypyridin

Vorstufe 2:

Synthese von 2-Hexyloxypyridin-5-boronsäure

250 mmol n-Butyllithium (1,6 m Lösung in n-Hexan) werden bei 0°C unter Feuchtigkeitsausschluß und Schutzgasatmosphäre zu einer Lösung von 250 mmol 5-Brom-2-hexyloxypyridin in 500 ml abs. Diethylether getropft. Man rührt 1 h bei dieser Temperatur nach und gibt anschließend 275 mmol Trimethylborat langsam zu. Nach einer weiteren Stunde Rühren bei 0°C wird eine Lösung aus 175 ml Wasser und 25 ml 37 proz. Salzsäure zugetropft. Man rührt 1 h bei Raumtemp., trennt anschließend die Phasen, extrahiert die wäßrige Phase mehrfach mit Dichlormethan und trocknet die vereinigten org. Extrakte mit Magnesiumsulfat. Die Lösemittel werden i. Vak. entfernt. Nach dem Trocknen im Feinvakuum wird das rotbraun gefärbte, hochviskose Rohprodukt ohne zusätzliche Reinigung weiter umgesetzt.

Auf analoge Weise wurden dargestellt:

2-Butyloxypyridin-5-boronsäure
2-Octyloxypyridin-5-boronsäure
2-Dodecyloxypyridin-5-boronsäure

Vorstufe 3:

Synthese von 5-Brom-2-(6-hexyloxypyridin-3-yl)-pyrimidin

Zu einer Lösung von 233 mmol 2,5-Dibrompyrimidin in 600 ml Toluol gibt man 233 mmol 2-Hexyloxypyridin-5-boronsäure, 300 ml Ethanol, eine Lösung von 466 mmol Natriumcarbonat in 150 ml Wasser und 2,3 mmol Tetrakis (triphenylphosphin)palladium(0). Es wird zum Sieden erhitzt, bis die Reaktion vollständig ist. Man trennt die org. Phase ab, extrahiert die wäßrige Phase mit Dichlormethan und trocknet die vereinigten org. Extrakte mit Natriumsulfat. Nach dem Entfernen der Lösemittel i. Vak. wird das Rohprodukt durch Säulenchromatographie an Kieselgel 60 mit Dichlormethan als Eluent abgetrennt und aus *n*-Heptan umkristallisiert. Man erhält 31 g (40%) eines farblosen Feststoffs, Schmp.: 103-106°C.

Vorstufe 4:

Synthese von 5-Brom-2-(6-butyloxypyridin-3-yl)-pyrimidin

Die Umsetzung von 109 mmol 2,5-Dibrompyrimidin, 109 mmol 2-Butyloxypyridin-5-boronsäure, 218 mmol Natriumcarbonat und 1,1 mmol Tetrakis(triphenylphosphin)palladium(0) in 200 ml Toluol, 100 ml Ethanol und 100 ml Wasser erfolgt analog der für Vorstufe 3 angegebenen Vorschrift. Nach entsprechender Reinigung erhält man 12,4 g (37%) eines farblosen Feststoffs, Schmp.: 134-138°C.

Vorstufe 5:

Synthese von 5-Brom-2-(6-octyloxypyridin-3-yl)-pyrimidin

Die Umsetzung von 115 mmol 2,5-Dibrompyrimidin, 115 mmol 2-Octyloxypyridin-5-boronsäure, 230 mmol Natriumcarbonat und 1,2 mmol Tetrakis(triphenylphosphin)palladium(0) in 280 ml Toluol, 140 ml Ethanol und 140 ml Wasser erfolgt analog der für Vorstufe 3 angegebenen Vorschrift. Nach entsprechender Reinigung erhält man 15,9 g (38%) eines farblosen Feststoffs, Schmp.: 99-101°C.

Vorstufe 6:

Synthese von 5-Brom-2-(6-dodecyloxypyridin-3-yl)-pyrimidin

Die Umsetzung von 35 mmol 2,5-Dibrompyrimidin, 27 mmol 2-Dodecyloxypyridin-5-boronsäure, 64 mmol Natriumcarbonat und 0,3 mmol Tetrakis(triphenylphosphin)palladium(0) in 210 ml Toluol, 135 ml Ethanol und 70 ml Wasser erfolgt analog der für Vorstufe 3 angegebenen Vorschrift. Nach entsprechender Reinigung erhält man 3,9 g (35%) eines farblosen Feststoffs.

Vorstufe 7:

Synthese von 2-Chlor-5-(6-hexyloxypyridin-3-yl)-pyrimidin

Die Umsetzung von 5-Brom-2-chlorpyrimidin und 2-Hexyloxypyridin-5-boronsäure erfolgt analog der für Vorstufe 3 angegebenen Vorschrift. Nach entsprechender Reinigung erhält man einen farblosen Feststoff.

Vorstufe 8:

Synthese von 5-(6-Butyloxypyridin-3-yl)-2-chlorpyrimidin

Die Umsetzung von 109 mmol 5-Brom-2-chlorpyrimidin, 109 mmol 2-Butyloxypyridin-5-boronsäure, 218 mmol Natriumcarbonat und 1,1 mmol Tetrakis(triphenylphosphin)palladium(0) in 200 ml Toluol, 100 ml Ethanol und 100 ml Wasser erfolgt analog der für Vorstufe 3 angegebenen Vorschrift. Nach entsprechender Reinigung erhält man 10,8 g (38%) eines farblosen Feststoffs, Schmp.: 117°C, Klp.: 130°C.

Vorstufe 9:

Synthese von 2-Chlor-5-(2,3-difluorphenyl)-pyrimidin

Die Umsetzung von 288 mmol 5-Brom-2-chlorpyrimidin, 288 mmol 2,3-Difluorphenylboronsäure, 576 mmol Natriumcarbonat und 5,8 mmol Tetrakis(triphenylphosphin)palladium(0) in 560 ml Toluol, 280 ml Ethanol und 280 ml Wasser erfolgt analog der für Vorstufe 3 angegebenen Vorschrift. Nach entsprechender Reinigung erhält man 37,7 g (58%) eines farblosen Feststoffs.

Vorstufe 10:

Synthese von 2-(4-Benzyloxy-2,3-difluorphenyl)-5-brompyrimidin

Die Umsetzung von 14 mmol 2,5-Dibrompyrimidin, 17 mmol 4-Benzyloxy-2,3-difluorphenylboronsäure, 34 mmol Natriumcarbonat und 0,14 mmol Tetrakis(triphenylphosphin)palladium(0) in 100 ml Toluol, 50 ml Ethanol und 50 ml Wasser erfolgt analog der für Vorstufe 3 angegebenen Vorschrift. Nach Reinigung durch Umkristallisation aus 2-Propanol erhält man 1,4 g (26%) eines farblosen Feststoffs, Schmp.: 138°C.

Vorstufe 11:

Synthese von 5-(4-Benzyloxyphenyl-2,3-difluor)-2-(6-hexyloxypyridin-3-yl)-pyrimidin

Die Umsetzung von 6 mmol 5-Brom-2-(6-hexyloxypyridin-3-yl)-pyrimidin, 6 mmol 4-Benzyloxy-2,3-difluorphenylboronsäure, 12 mmol Natriumcarbonat, 0,06 mmol Bis(dibenzylidenaceton)palladium(0) und 0,12 mmol Triphenylphosphin in 50 ml Toluol, 25 ml Ethanol und 25 ml Wasser erfolgt analog der für Beispiel 1a) angegebenen Vorschrift. Nach entsprechender chromatographischer Reinigung erhält man 1,4 g (49%) eines farblosen Feststoffs, Schmp.: 125°C, Klp.: 174°C.

Vorstufe 12:

Synthese von 5-(4-Benzyloxy-2,3-difluorphenyl)-2-(6-dodecyloxypyridin-3-yl)-pyrimidin

Die Umsetzung von 6 mmol 5-Brom-2-(6-dodecyloxypyridin-3-yl)-pyrimidin, 6 mmol 4-Benzyloxy-2,3-difluorphenylboronsäure, 12 mmol Natriumcarbonat, 0,06 mmol Bis(dibenzylidenaceton)palladium(0) und 0,12 mmol Triphenylphosphin in 50 ml Toluol, 25 ml Ethanol und 25 ml Wasser erfolgt analog der für Beispiel 1a) angegebenen Vorschrift. Nach entsprechender chromatographischer Reinigung erhält man 1,4 g (42%) eines farblosen Feststoffs, Schmp.: 115°C, Klp.: 172°C.

Vorstufe 13:

Synthese von 2-(4-Benzyloxy-2,3-difluorphenyl)-5-(6-octyloxypyridin-3-yl)-pyrimidin Die Umsetzung von 35 mmol 2-(4-Benzyloxy-2,3-difluorphenyl)-5-brompyrimidin, 44 mmol 2-Octyloxypyridin-5-boronsäure, 70 mmol Natriumcarbonat und 0,4 mmol Tetrakis(triphenylphosphin)palladium(0) in 120 ml Toluol, 60 ml Ethanol und 60 ml Wasser erfolgt analog der für Beispiel 1a) angegebenen Vorschrift. Nach entsprechender chromatographischer Reinigung und Umkristallisation aus Essigsäureethylester erhält man 10,7 g (61%) farblose Kristalle, Schmp.: 120°C, Klp.: 182-183°C.

Vorstufe 14:

Synthese von 5-(2,3-Difluor-4-hydroxyphenyl)-2-(6-hexyloxypyridin-3-yl)-pyrimidin

Zu einer Lösung von 14 mmol 5-(4-Benzyloxy-2,3-difluorphenyl)-2-(6-hexyloxypyridin-3-yl)-pyrimidin in 200 ml THF gibt man 1,5 g 10 proz. Palladium/Kohle und 0,1 g 4-Toluolsulfonsäure. In einer geeigneten Apparatur rührt man bei 50°C unter Wasserstoff, bis dessen Aufnahme beendet ist, filtriert vom Katalysator und entfernt das Lösemittel im Vakuum. Nach Reinigung durch Umkristallisation aus Acetonitril erhält man 4,0 g (73%) eines farblosen Feststoffs, Schmp.: 150-155°C.

Vorstufe 15:

Synthese von 5-(2,3-Difluor-4-hydroxyphenyl)-2-(6-dodecyloxypyridin-3-yl)-pyrimidin

Die Hydrierung von 2,5 mmol 5-(4-Benzyloxy-2,3-difluorphenyl)-2-(6-dodecyloxypyridin-3-yl)-pyrimidin in 50 ml THF erfolgt analog der für Vorstufe 14 angegebenen Vorschrift. Man erhält 0,7 g (60%) eines farblosen Feststoffs, Schmp.: 111-118°C.

Vorstufe 16:

Synthese von 2-(2,3-Difluor-4-hydroxyphenyl)-5-(6-octyloxypyridin-3-yl)-pyrimidin

Die Hydrierung von 21 mmol 2-(4-Benzyloxy-2,3-difluorphenyl)-5-(6-octyloxypyridin-3-yl)-pyrimidin in 200 ml THF erfolgt analog der für Vorstufe 14 angegebenen Vorschrift. Man erhält 8,2 g (93%) eines farblosen Feststoffs, Schmp.: 130-132°C.

Beispiel 1:

Synthese von 5-(2,3-Difluor-4-octyloxyphenyl)-2-(6-hexyloxypyridin-3-yl)-pyrimidin

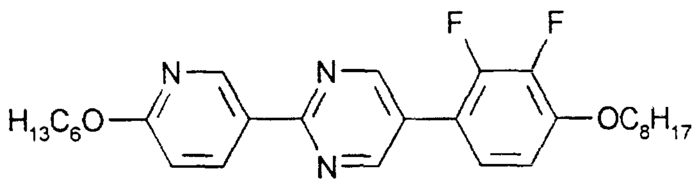

a)
Zu einer Lösung von 58 mmol 5-Brom-2-(6-hexyloxypyridin-3-yl)-pyrimidin in 360 ml Toluol gibt man 58 mmol 2,3-Difluor-4-octyloxyphenylboronsäure, 180 ml Ethanol, eine Lösung von 116 mmol Natriumcarbonat in 90 ml Wasser und 0,6 mmol Tetrakis(triphenylphosphin)palladium(0). Es wird zum Sieden erhitzt, bis die Reaktion vollständig ist. Man trennt die org. Phase ab, extrahiert die wäßrige Phase mit Dichlormethan und trocknet die vereinigten org. Extrakte mit Natriumsulfat. Nach dem Entfernen der Lösemittel i. Vak. wird das Rohprodukt durch Säulenchromatographie an Kieselgel 60 mit Dichlormethan/Essigsäureethylester 20:1 (v/v) als Eluent abgetrennt und aus Methanol umkristallisiert. Man erhält 11g (63%) farblose Kristalle, X 82,5 $S_C$ 130 $S_A$ 160 I.

b)
Die Veretherung von 12 mmol 5-(2,3-Difluor-4-hydroxyphenyl)-2-(6-hexyloxypyridin-3-yl)-pyrimidin mit 13,2 mmol 1-Bromoctan in 100 ml DMF erfolgt analog der für Beispiel 3 angegebenen Vorschrift. Das Rohprodukt wird an Kieselgel 60 mit Dichlormethan als Eluent chromatographiert sowie aus n-Heptan und Toluol umkristallisiert. Man erhält 3,6 g (61%) farblose Kristalle, deren analytische Daten mit denen der nach 1a) erhaltenen Verbindung übereinstimmen.

Beispiel 2:

Synthese von 5-(2,3-Difluor-4-octyloxyphenyl)-2-(6-dodecyloxypyridin-3-yl)-pyrimidin

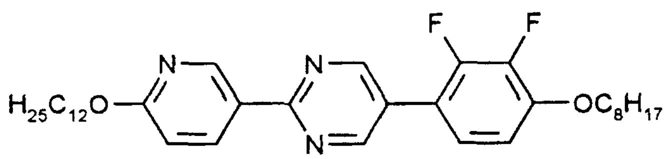

Die Veretherung von 1,3 mmol 5-(2,3-Difluor-4-hydroxyphenyl)-2-(6-dodecyloxypyridin-3-yl)-pyrimidin mit 1,7 mmol 1-Bromoctan in 80 ml DMF erfolgt analog der für Beispiel 3 angegebenen Vorschrift. Nach chromatographischer Reinigung an Kieselgel 60 mit Dichlormethan als Eluent und Umkristallisation aus Methanol erhält man 380 mg (51%) farblose Kristalle, X 67 $X_1$ 76 $S_C$ 118-123 $S_A$ 147 I.

Beispiel 3:

Synthese von 2-(2,3-Difluor-4-octyloxyphenyl)-5-(6-octyloxypyridin-3-yl)-pyrimidin

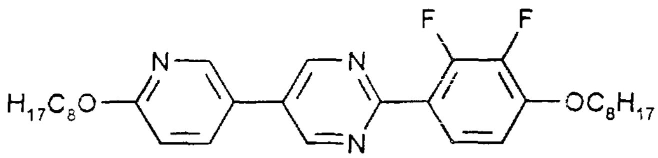

Zu einer Suspension von 2,7 mmol Natriumhydrid (80 proz. in Mineralöl) in 5 ml trockenem DMF wird bei Raumtemp. und unter Schutzgas eine Lösung von 2,4 mmol 2-(2,3-Difluor-4-hydroxyphenyl)-5-(6-octyloxypyridin-3-yl)-pyrimidin in 10 ml DMF getropft. Man rührt 30 min nach und gibt anschließend 2,7 mmol 1-Bromoctan, gelöst in 10 ml DMF, langsam zu. Nach 20 h Rühren bei Raumtemp. gibt man die Reaktionsmischung auf 100 ml Eis/Wasser, extrahiert mehrfach mit Dichlormethan, wäscht die vereinigten org. Extrakte mit ges. Natriumchloridlösung und trocknet sie mit Magnesiumsulfat. Nach dem Entfernen des Lösemittels i. Vak. wird das Rohprodukt an Kieselgel 60 mit Dichlormethan/ Essigsäureethylester 20:1 (v/v) als Eluent chromatographiert sowie aus Acetonitril umkristallisiert. Man erhält 0,77 g (61%) farblose Kristalle, X 89 $S_3$ 73,5 $S_C$ 164 $S_A$ 167 I.

Beispiel 4:

Synthese von 2-(2,3-Difluor-4-hexyloxyphenyl)-5-(6-octyloxypyridin-3-yl)-pyrimidin

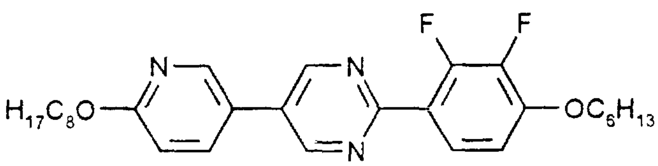

Die Veretherung von 2,4 mmol 2-(2,3-Difluor-4-hydroxyphenyl)-5-(6-octyloxypyridin-3-yl)-pyrimidin mit 2,7 mmol 1-Bromhexan erfolgt analog der für Beispiel 3 angegebenen Vorschrift. Nach entsprechender Reinigung erhält man 0,88 g (73%) farblose Kristalle, X 81,5 $S_3$ 75,5 $S_C$ 165,5 $S_A$ 174 I.

Beispiel 5:

Synthese 2-(4-Butoxy-2,3-difluorphenyl)-5-(6-octyloxypyridin-3-yl)-pyrimidin

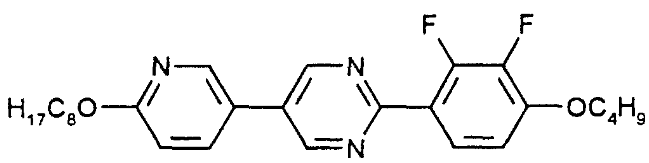

Die Veretherung von 2,4 mmol 2-(2,3-Difluor-4-hydroxyphenyl)-5-(6-octyloxypyridin-3-yl)-pyrimidin mit 2,7 mmol 1-Brombutan erfolgt analog der für Beispiel 3 angegebenen Vorschrift. Nach entsprechender Reinigung erhält man 0,76 g (69%) farblose Kristalle, X 85 $S_C$ 156 $S_A$ 180 1.

Beispiel 6:

Synthese von 2-(4-Ethoxy-2,3-difluorphenyl)-5-(6-octyloxypyridin-3-yl)-pyrimidin

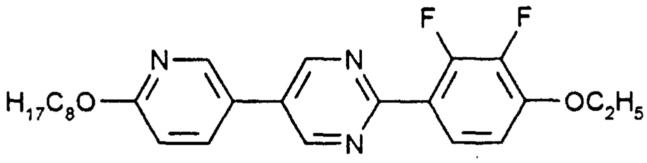

Die Veretherung von 2,8 mmol 2-(2,3-Difluor-4-hydroxyphenyl)-5-(6-octyloxypyridin-3-yl)-pyrimidin mit 3,0 mmol 1-Bromethan erfolgt analog der für Beispiel 3 angegebenen Vorschrift. Nach entsprechender Reinigung erhält man 0,62 g (51%) farblose Kristalle, X 111 $S_C$ 122,5 $S_A$ 185 I.

Beispiel 7:

Synthese von 2-[2,3-Difluor-4-(2-(S)-fluordecyloxy)-phenyl]-5-(6-octyloxypyridin-3-yl)-pyrimidin

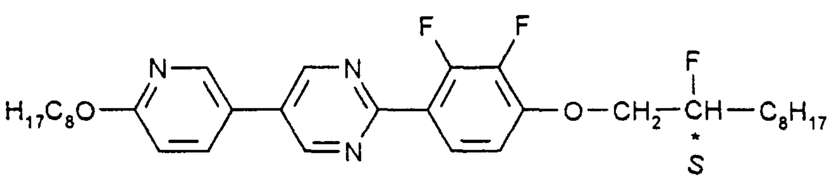

Zu 3,2 mmol Triphenylphosphin in 20 ml THF tropft man bei 0°C 3,2 mmol Azodicarbonsäurediethylester und rührt 20 min bei Raumtemp. nach. Anschließend gibt man 2,4 mmol 2-(2,3-Difluor-4-hydroxyphenyl)-5-(6-octyloxypyridin-3-yl)-pyrimidin und 2,4 mmol 2-(S)-Fluordecanol zu und läßt die Reaktionsmischung über Nacht stehen. Nach dem Entfernen des Lösemittels i. Vak. wird das Rohprodukt an Kieselgel 60 mit Dichlormethan/Essigsäureethylester 20:1 (v/v) als Eluent chromatographiert sowie aus Acetonitril umkristallisiert. Man erhält 1,2 g (86%) farblose Kristalle, X 97 $S_C^*$ 150,5 $S_A$ 161,5 I.

Beispiel 8:

Synthese von 2-[4-(3-Butyl-(2S,3S)-oxiranylmethoxy)-2,3-difluorphenyl]-5-(6-octyloxypyridin-3-yl)-pyrimidin

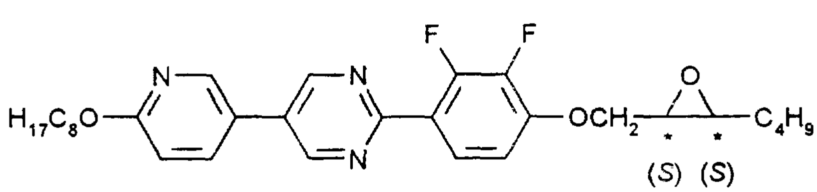

Die Veretherung von 2,4 mmol 2-(2,3-Difluor-4-hydroxyphenyl)-5-(6-octyloxypyridin-3-yl)-pyrimidin mit 2,4

mmol (1S,2S)-1-Butyl-2-(hydroxymethyl)oxiran erfolgt analog der für Beispiel 7 angegebenen Vorschrift. Nach entsprechender Reinigung erhält man 0,49 g (39%) farblose Kristalle, X 84,5 $S_C$* 166 $S_A$ 174 I.

Beispiel 9:

Synthese von Heptansäure 2,3-difluor-4-[5-(6-octyloxypyridin-3-yl)-pyrimidin-2-yl]-phenyl ester

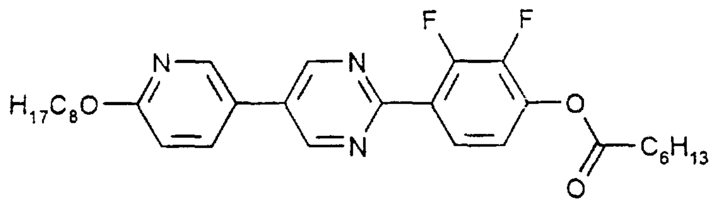

2,7 mmol DCC werden bei Raumtemp. in 50 ml Dichlormethan vorgelegt und 2,4 mmol 2-(2,3-Difluor-4-hydroxyphenyl)-5-(6-octyloxypyridin-3-yl)-pyrimidin, 2,7 mmol Heptansäure und 0,24 mmol DMAP zugegeben. Die Reaktionsmischung läßt man über Nacht unter Lichtausschluß stehen, filtriert anschließend vom ausgefallenen Dicyclohexylharnstoff und entfernt das Lösemittel im Vakuum. Das Rohprodukt wird an Kieselgel 60 mit Dichlormethan/Essigsäureethylester 20:1 (v/v) als Eluent chromatographiert sowie aus Acetonitril umkristallisiert. Man erhält 1,0 g (84%) farblose Kristalle, X 88 $S_C$ 166 $S_A$ 167,51.

Beispiel 10:

Synthese von Kohlensäure-2,3-difluor-4-[5-(6-octyloxypyridin-3-yl)-pyrimidin-2-yl]-phenyl ester ethyl ester

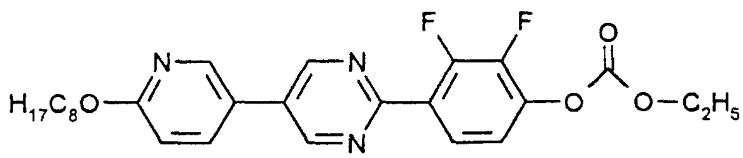

2,4 mmol 2-(2,3-Difluor-4-hydroxyphenyl)-5-(6-octyloxypyridin-3-yl)-pyrimidin werden in 20 ml THF vorgelegt und auf 0°C gekühlt. Man tropft zunächst 2,7 mmol Triethylamin, anschließend 2,7 mmol Chlorkohlensäureethylester in 20 ml THF zu und rührt 1 h bei 0°C. Die Reaktionsmischung steht über Nacht bei Raumtemperatur. Man filtriert, entfernt das Lösemittel i. Vak. und chromatographiert das Rohprodukt an Kieselgel 60 mit Dichlormethan/Essigsäureethylester 20:1 (v/v) als Eluent. Nach Umkristallisation aus Acetonitril erhält man 0,75 g (64%) farblose Kristalle, X 74 $S_C$ 92 $S_A$ 165 N 172,5 I.

Beispiel 11:

Synthese von 5-(2,3-Difluor-4-octylphenyl)-2-(6-hexyloxypyridin-3-yl)-pyrimidin

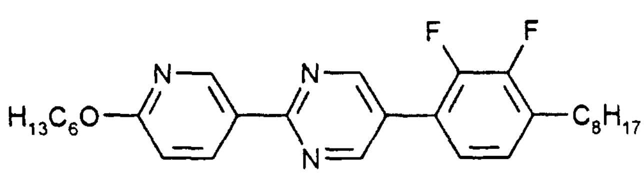

Die Umsetzung von 2 mmol 5-Brom-2-(6-hexyloxypyridin-3-yl)-pyrimidin, 2,2 mmol 2,3-Difluor-4-octylphenylboronsäure, 4 mmol Natriumcarbonat und 0,02 mmol Tetrakis(triphenylphosphin)palladium(0) in 14 ml Toluol, 7 ml Ethanol und 7 ml Wasser erfolgt analog der für Beispiel 1a) angegebenen Vorschrift. Das Rohprodukt wird durch Säulenchromatographie an Kieselgel 60 mit n-Heptan/ Essigsäureethylester 9:1 (v/v) als Eluent abgetrennt und aus Acetonitril umkristallisiert. Man erhält 0,77 g (80%) farblose Kristalle, X 104 $S_C$ 94 $S_A$ 129 I.

Beispiel 12:

Synthese von 5-(4-Decyl-2,3-difluorphenyl)-2-(6-hexyloxypyridin-3-yl)- pyrirnidin

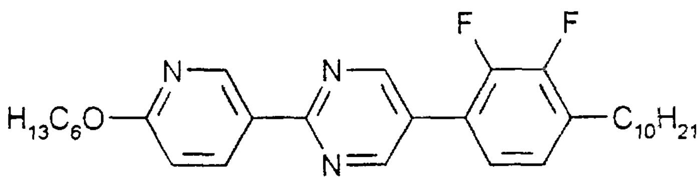

Die Umsetzung von 2 mmol 5-Brom-2-(6-hexyloxypyridin-3-yl)-pyrimidin, 2 mmol 4-Decyl-2,3-difluorphenylboronsäure, 4 mmol Natriumcarbonat und 0,02 mmol Tetrakis(triphenylphosphin)palladium(0) in 8 ml Toluol, 3 ml Ethanol und 3 ml Wasser erfolgt analog der für Beispiel 1a) angegebenen Vorschrift. Das Rohprodukt wird durch Säulenchromatographie an Kieselgel 60 mit Essigsäureethylester als Eluent abgetrennt und aus Acetonitril umkristallisiert. Man erhält 0,4 g (39%) farblose Kristalle, X 96 $S_A$ 127 I.

Beispiel 13:

Synthese von 2-(6-Butyloxypyridin-3-yl)-5-(2,3-difluor-4-octylphenyl)-pyrimidin

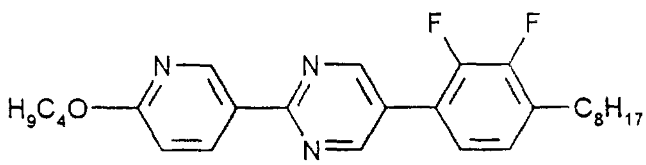

Die Umsetzung von 1,6 mmol 5-Brom-2-(6-butyloxypyridin-3-yl)-pyrimidin, 1,8 mmol 2,3-Difluor-4-octylphenylboronsäure, 3,2 mmol Natriumcarbonat und 0,02 mmol Tetrakis(triphenylphosphin)palladium(0) in 10 ml Toluol, 5 ml Ethanol und 5 ml Wasser erfolgt analog der für Beispiel 1a) angegebenen Vorschrift. Das Rohprodukt wird durch Säulenchromatographie an Kieselgel 60 mit Heptan/Essigsäureethylester 9:1 (v/v) als Eluent abgetrennt und aus Acetonitril umkristallisiert. Man erhält 0,53 g (73%) farblose Kristalle, X 113 $S_A$ 132,5 I.

Beispiel 14:

Synthese von 5-(2,3-Difluor-4-octylphenyl)-2-(6-octyloxypyridin-3-yl)-pyrimidin

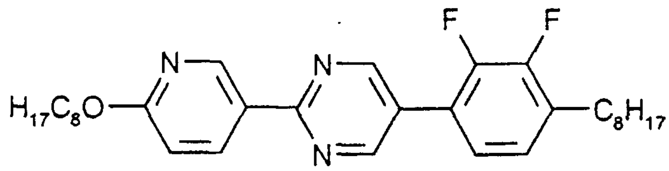

Die Umsetzung von 3,5 mmol 5-Brom-2-(6-octyloxypyridin-3-yl)-pyrimidin, 3,85 mmol 2,3-Difluor-4-octylphenylboronsäure, 7 mmol Natriumcarbonat und 0,04 mmol Tetrakis(triphenylphosphin)palladium(0) in 20 ml Toluol, 10 ml Ethanol und 10 ml Wasser erfolgt analog der für Beispiel 1 a) angegebenen Vorschrift. Das Rohprodukt wird durch Säulenchromatographie an Kieselgel 60 mit Heptan/Essigsäureethylester 9:1 (v/v) als Eluent abgetrennt und aus Acetonitril umkristallisiert. Man erhält 1,5 g (83%) farblose Kristalle, X 105 $S_C$ 104 $S_A$ 127 I.

Beispiel 15:

Synthese von 5-(2,3-Difluorphenyl)-2-(6-hexyloxypyridin-3-yl)-pyrimidin

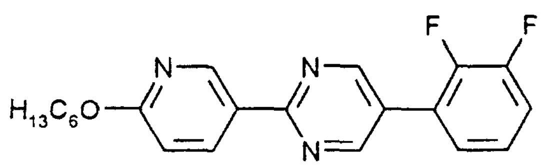

Die Umsetzung von 2-Chlor-5-(2,3-difluorphenyl)-pyrimidin mit 2-Hexyloxypyridin-5-boronsäure erfolgt analog der für Beispiel 1a) angegebenen Vorschrift. Nach chromatographischer Reinigung an Kieselgel 60 mit Dichlormethan/Essigsäureethylester 95:5 (v/v) als Eluent und Umkristallisation aus Acetonitril erhält man farblose Kristalle, Schmp.: 110,5°C.

Beispiel 16:

Synthese von 2-(2,3-Difluor-4-octylphenyl)-5-(6-hexyloxypyridin-3-yl)-pyrimidin

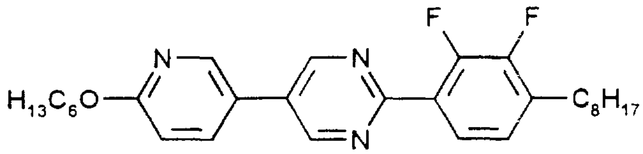

Die Umsetzung von 2,5 mmol 2-Chlor-5-(6-hexyloxypyridin-3-yl)-pyrimidin, 2,5 mmol 2,3-Difluor-4-octylphenylboronsäure, 5 mmol Natriumcarbonat und 0,025 mmol Tetrakis(triphenylphosphin)palladium(0) in 8 ml Toluol, 4 ml Ethanol und 4 ml Wasser erfolgt analog der für Beispiel 1a) angegebenen Vorschrift. Das Rohprodukt wird durch Säulenchromatographie an Kieselgel 60 mit Dichlormethan/Essigsäureethylester 98:2 (v/v) als Eluent abgetrennt und aus Acetonitril umkristallisiert. Man erhält 0,47 g (39%) farblose Kristalle, X 94,5 $S_C$ 125,5 $S_A$ 156 I.

Beispiel 17:

Synthese von 5-(6-Butyloxypyridin-3-yl)-2-(2,3-difluor-4-octylphenyl)-pyrimidin

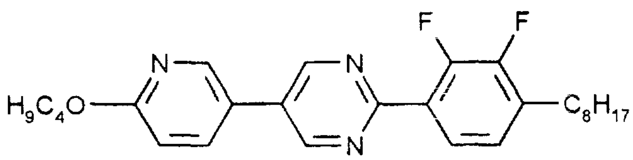

Die Umsetzung von 2 mmol 5-(6-butyloxypyridin-3-yl)-2-chlorpyrimidin, 2,2 mmol 2,3-Difluor-4-octylphenylboronsäure, 4 mmol Natriumcarbonat und 0,02 mmol Tetrakis(triphenylphosphin)palladium(0) in 10 ml Toluol, 5 ml Ethanol und 5 ml Wasser erfolgt analog der für Beispiel 1a) angegebenen Vorschrift. Das Rohprodukt wird durch Säulenchromatographie an Kieselgel 60 mit Heptan/Essigsäureethylester 9:1 (v/v) als Eluent abgetrennt und aus Acetonitril umkristallisiert. Man erhält 0,42 g (46%) farblose Kristalle, X 84 Sc 73 $S_A$ 162 I.

Anwendungsbeispiele

Eine Grundmischung A enthält die folgenden Komponenten:

| | Gew.-% |
|---|---|
| 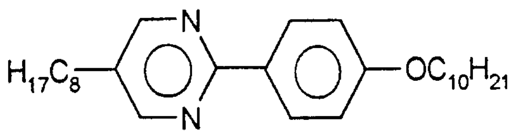 | 15,44 |
| 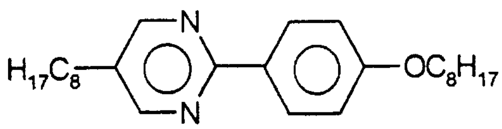 | 19,42 |
| 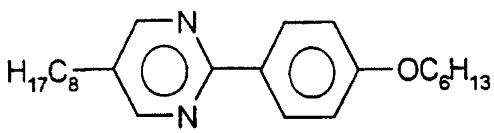 | 20,10 |
| 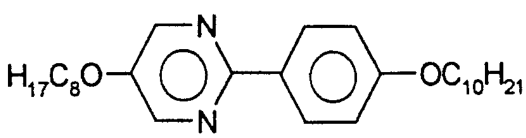 | 12,61 |
| 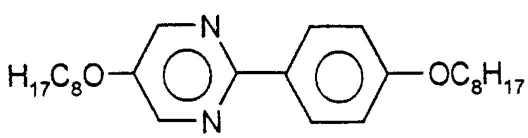 | 6,50 |
| 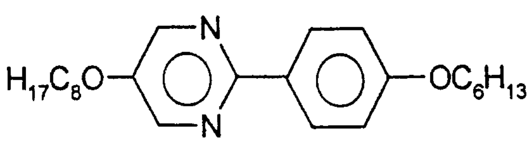 | 13,11 |
| 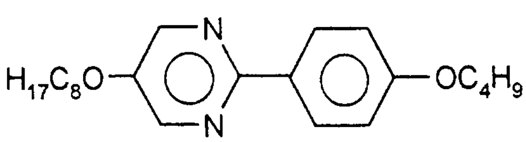 | 12,82 |

Eine FLC-Mischung B enthält die folgenden Komponenten:

| | Gew.-% |
|---|---|
| Grundmischung A | 78 |
| 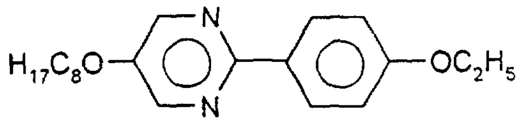 | 10 |
| 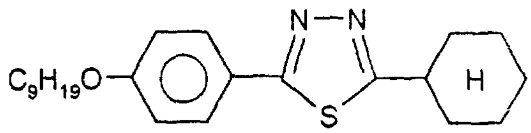 | 10 |
| 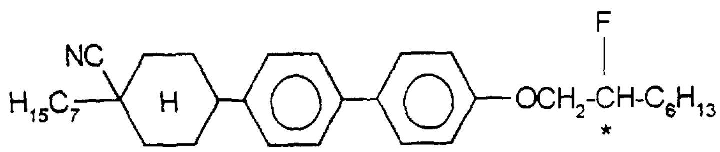 | 2 |

Anwendungsbeispiel 1

Eine FLC-Mischung C enthält die folgenden Komponenten:

| | Gew.-% |
|---|---|
| Grundmischung A | 68 |
| 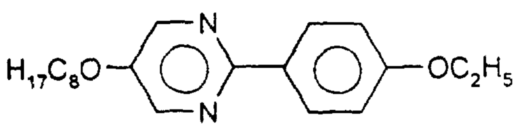 | 10 |
| 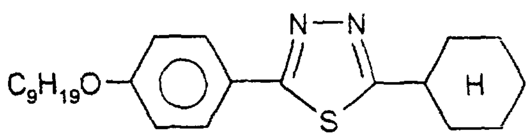 | 10 |
| 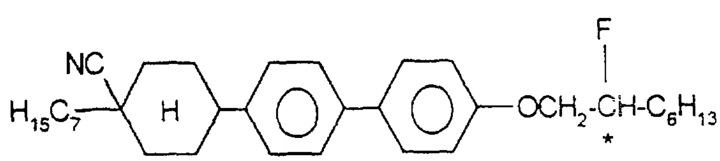 | 2 |
| 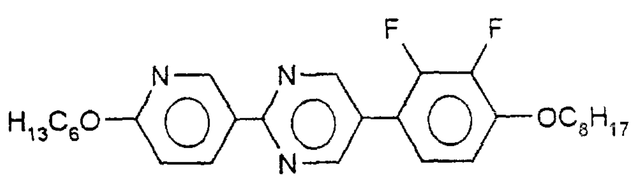 (Substanz aus Beispiel 1) | 10 |

Anwendungsbeispiel 2

Eine FLC-Mischung D enthält die folgenden Komponenten:

| | Gew.-% |
|---|---|
| Grundmischung A | 68 |
| 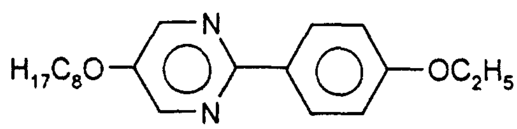 | 10 |
| 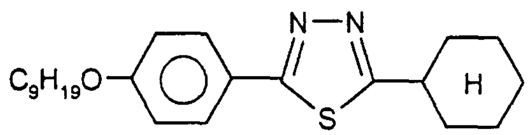 | 10 |
| 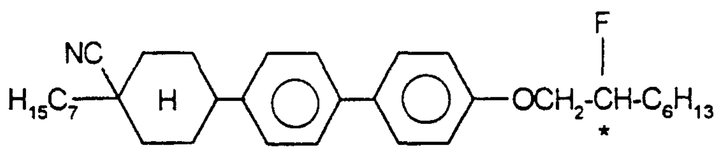 | 2 |
| 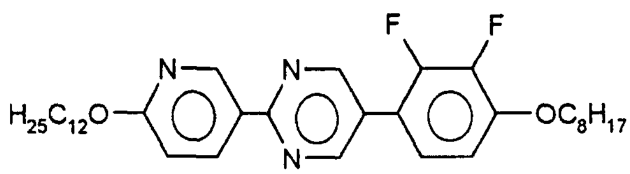 (Substanz aus Beispiel 2) | 10 |

Eine FLC-Mischung E enthält die folgenden Komponenten:

| | Gew.-% |
|---|---|
| Grundmischung A | 68 |
| 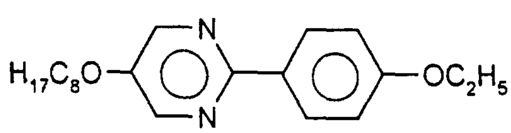 | 10 |
| 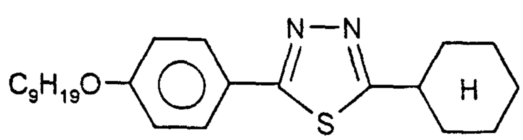 | 10 |

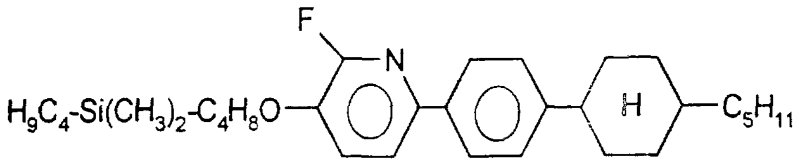 10

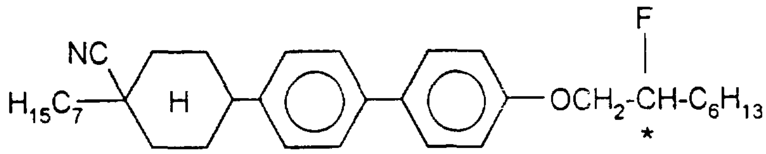 2

Anwendungsbeispiel 3

Eine FLC-Mischung F enthält die folgenden Komponenten:

Gew.-%

Grundmischung A 58

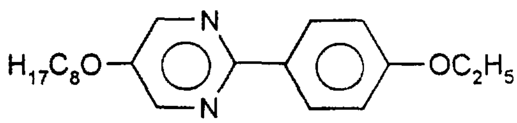

10

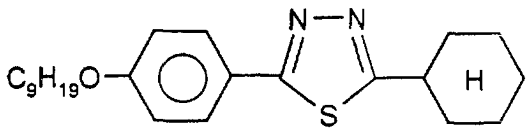

10

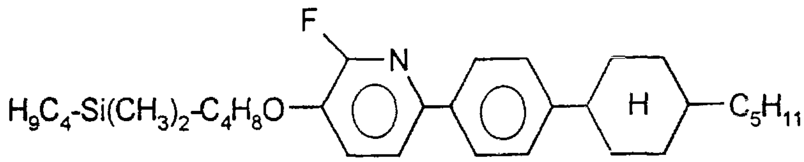

10

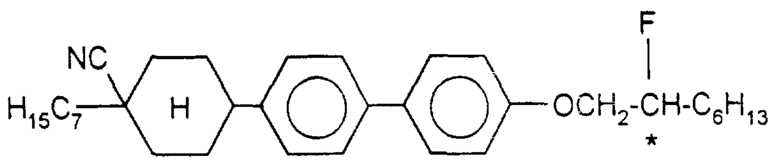

2

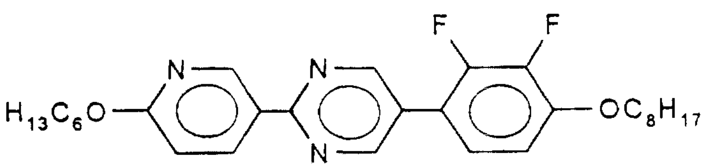

10

(Substanz aus Beispiel 1)

Tabelle:

| Phasen und elektrooptische Eigenschaften: | | | | | | | | | | | |
|---|---|---|---|---|---|---|---|---|---|---|---|
| Mischung: | Ta/c | Ta/n | Tn/i | $V_{min}$ (Mo) | $T_{min}$ (Mo) | $V_{min}$ (M3) | $T_{min}$ (M3) | $V_{min}$ (JA) | $T_{min}$ (JA) | 2e (5v) | 2e (10v) |
| B | 62 | 75 | 82 | 65 | 18 | 55 | 63 | 60 | 18 | 16 | 23 |
| C (Anw. Bsp.1) | 61 | 77 | 86 | 53 | 20 | 46 | 7,5 | 53 | 20 | 20 | 28 |
| D (Anw. Bsp.2) | 66 | 78 | 83 | 55 | 24 | 51 | 9,1 | 58 | 26 | 18 | 27 |
| D | | | | 60 | 17 | 51 | 6,8 | 60 | 17 | 15 | 28 |
| E (Anw. Bsp.3) | | | | 40 | 22 | 40 | 6,5 | 42 | 27 | 19 | 30 |

Dabei bedeuten:

| | |
|---|---|
| Ta/c: | Phasenübergangstemperatur $S_C \rightarrow S_A$[°C] |
| Ta/n: | " $S_A \rightarrow N$ [°C] |
| Tn/i: | " $N \rightarrow$ isotrop [°C] |
| $V_{min}$, $T_{min}$: | Minimum der V,T-Kurve |
| Mo: | Monopolar-Schema |
| M3: | Malvern3-Schema : (J.R. Hughes, E.P. Raynes, Liq.Cryst. 1993, 13, 597; errata corrige, 1993, 15, 281.) |
| JA: | Joers-Alvey-Schema : J.C. Jones, M.J. Towler, J.R. Hughes, Display 1993, 14, 86.) |
| 2e: | Schaltwinkel [°] |

Mischungen, die eine erfindungsgemäße Verbindung enthalten, weisen einen erheblich niedrigeren Wert für die Einsatzspannung im Minimum der V,T-Kurve auf.

## Patentansprüche

1. Difluorphenylpyrimidyipyridin-Derivat der Formel (I)

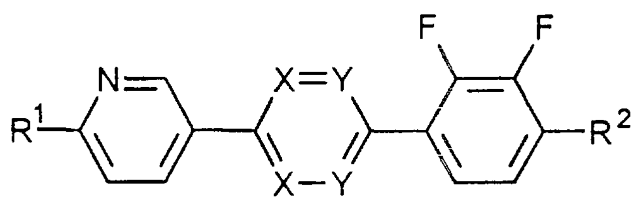

(I)

in der die Symbole folgende Bedeutungen haben:

X ist N und Y ist CH oder X ist CH und Y ist N;
$R^1$, $R^2$ sind gleich oder verschieden

a) eine unverzweigte oder verzweigte Alkylkette mit 1 bis 20 C-Atomen, wobei
aa) eine oder mehrere nicht benachbarte und nicht terminale $CH_2$-Gruppen durch -O-, -CO-O-, -O-CO-, -O-CO-O-, $-Si(CH_3)_2-$ ersetzt sein können, undloder
ab) ein oder mehrere H-Atome durch F ersetzt sein können, und/oder
ac) die terminale $CH_3$-Gruppe durch eine der folgenden chiralen Gruppen (optisch aktiv oder racemisch) ersetzt sein kann:

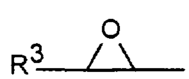

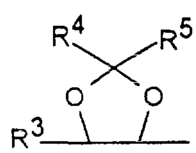

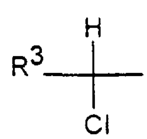

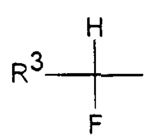

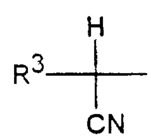

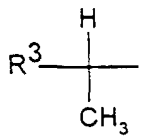

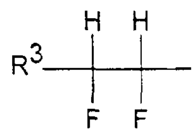

b) Wasserstoff, wobei nur einer der beiden Reste $R^1$, $R^2$ H sein darf,

mit der Maßgabe, daß $R^1$ nicht über -CO-O- oder -O-CO-O- an den Pyridinring gebunden sein darf; $R^3$, $R^4$, $R^5$ sind gleich oder verschieden Wasserstoff oder ein geradkettiger oder verzweigter Alkylrest mit 1-16 C-Atomen (mit oder ohne asymmetrisches C-Atom), wobei auch eine oder mehrere nicht benachbarte, nicht terminale $CH_2$-Gruppen durch -O- ersetzt sein können, und/oder wobei ein oder mehrere H-Atome des Alkylrestes durch -F substituiert sein können; $R^4$ und $R^5$ können zusammen auch $-(CH_2)_4-$ oder $-(CH_2)_5-$ sein, wenn sie an ein Dioxolan-System gebunden sind.

**2.** Difluorphenylpyrimidylpyridin-Derivat gemäß Anspruch 1, dadurch gekennzeichnet, daß die Symbole in der Formel (I) folgende Bedeutungen haben:

$R^1$, $R^2$ sind gleich oder verschieden

a) eine unverzweigte oder verzweigte Alkylkette mit 1 bis 16 C-Atomen, wobei
aa) eine oder mehrere nicht benachbarte und nicht terminale $CH_2$-Gruppen durch -O-, -CO-O-, -O-CO-, -O-CO-O-, $-Si(CH_3)_2-$ ersetzt sein können, und/oder
ab) ein oder mehrere H-Atome durch F ersetzt sein können, und/oder
ac) die terminale $CH_3$-Gruppe durch eine der folgenden chiralen Gruppen (optisch aktiv oder racemisch) ersetzt sein kann:

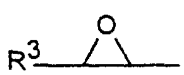

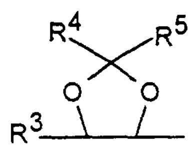

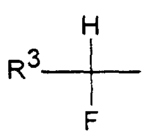

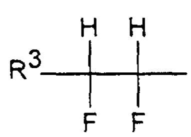

b) Wasserstoff, wobei nur einer der beiden Reste $R^1$, $R^2$ H sein darf,

mit der Maßgabe, daß $R^1$ nicht über -CO-O- oder -O-CO-O- an den Pyridinring gebunden sein darf;

$R^3$, $R^4$, $R^5$ sind gleich oder verschieden Wasserstoff oder ein geradkettiger oder verzweigter Alkylrest mit 1-13 C-Atomen, wobei auch eine oder mehrere nicht benachbarte, nicht terminale $CH_2$-Gruppen durch -O- ersetzt sein können und/oder wobei ein oder mehrere H-Atome des Alkylrestes durch -F substituiert sein können; $R^4$ und $R^5$ können zusammen auch $-(CH_2)_4-$ oder $-(CH_2)_5-$ sein, wenn sie an ein Dioxolan-System gebunden sind.

**3.** Difluorphenylpyrimidylpyridin-Derivat gemäß Anspruch 2, dadurch gekennzeichnet, daß die Symbole in der Formel (I) folgende Bedeutungen haben:

$R^1$, $R^2$ sind gleich oder verschieden

a) eine unverzweigte oder verzweigte Alkylkette mit 1 bis 12 C-Atomen, wobei
aa) eine oder mehrere nicht benachbarte und nicht terminale $CH_2$-Gruppen durch -O-, -CO-O-, -O-CO-, -O-CO-O- ersetzt sein können, und/oder
ab) ein oder mehrere H-Atome durch F ersetzt sein können, und/oder
ac) die terminale $CH_3$-Gruppe durch eine der folgenden chiralen Gruppen (optisch aktiv oder racemisch) ersetzt sein kann:

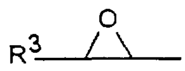

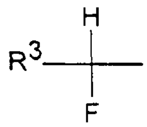

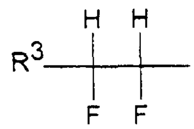

b) Wasserstoff, wobei nur einer der beiden Reste $R^1$, $R^2$ H sein darf,
mit der Maßgabe, daß $R^1$ nicht über -CO-O- oder -O-CO-O- an den Pyridinring gebunden sein darf;

$R^3$ ist gleich oder verschieden Wasserstoff oder ein geradkettiger oder verzweigter Alkylrest mit 1 bis 10 C-Atomen, wobei auch eine oder zwei nicht benachbarte, nicht terminale $CH_2$-Gruppen durch -O- ersetzt sein können.

**4.** Difluorphenylpyrimidylpyridin-Derivat gemäß einem oder mehreren der vorhergehenden Ansprüche, dadurch gekennzeichnet, daß die Symbole in der Formel (I) folgende Bedeutungen haben:

$R^1$, $R^2$ sind gleich oder verschieden

a) eine unverzweigte oder verzweigte Alkylkette mit 1 bis 12 C-Atomen, wobei
aa) eine oder mehrere nicht benachbarte und nicht terminale $CH_2$-Gruppen durch -O-, -CO-O-, -O-CO-, -O-CO-O- ersetzt sein können, und/oder
ab) die terminale $CH_3$-Gruppe durch eine der folgenden chiralen Gruppen (optisch aktiv oder racemisch) ersetzt sein kann:

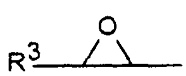

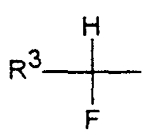

b) Wasserstoff, wobei nur einer der beiden Reste $R^1$, R2 H sein darf,

mit der Maßgabe, daß $R^1$ nicht über -CO-O- oder -O-CO-O- an den Pyridinring gebunden sein darf;

$R^3$ ist gleich oder verschieden Wasserstoff oder ein geradkettiger oder verzweigter Alkylrest mit 1 bis 10 C-Atomen, wobei auch eine oder zwei nicht benachbarte, nicht terminale $CH_2$-Gruppen durch -O- ersetzt sein können.

**5.** Verwendung von Difluorphenylpyrimidylpyridin-Derivaten gemäß einem oder mehreren der Ansprüche 1 bis 4 als Komponenten flüssigkristalliner Mischungen.

**6.** Flüssigkkristallmischung, enthaltend ein oder mehrere Difluorphenylpyrimidylpyridin-Derivate gemäß einem oder mehreren der Ansprüche 1 bis 4.

**7.** Flüssigkristallmischung gemäß Anspruch 6, dadurch gekennzeichnet, daß sie ferroelektrisch ist.

**8.** Flüssigkristallmischung gemäß Anspruch 6 und/oder 7, dadurch gekennzeichnet, daß sie 0,01 bis 80 Gew.-% an einer oder mehreren Difluorphenylpyrimidylpyridin-Derivaten der Formel (I) enthält.

**9.** Ferroelektrische Schalt- und/oder Anzeigevorrichtung, enthaltend eine ferroelektrische Flüssigkristallmischung gemäß Anspruch 7 und/oder 8.

**10.** Ferroelektrische Schalt- und/oder Anzeigevorrichtung gemäß Anspruch 9, dadurch gekennzeichnet, daß sie im $TV_{min}$-Mode betrieben wird.

**Claims**

**1.** A difluorophenylpyrimidylpyridine derivative of the formula (I)

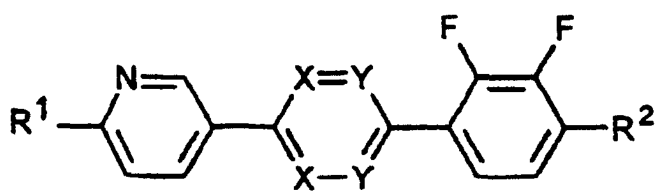

(I)

in which the symbols are defined as follows:

X is N and Y is CH or X is CH and Y is N;
$R^1$ and $R^2$ are identical or different and are

a) an unbranched or branched alkyl chain having 1 to 20 carbon atoms, where
aa) one or more non-adjacent and non-terminal $CH_2$ groups may be replaced by -O-, -CO-O-, -O-CO-, -O-CO-O- or $-Si(CH_3)_2-$, and/or
ab) one or more H atoms may be replaced by F, and/or
ac) the terminal $CH_3$ group may be replaced by one of the following chiral groups (optically active or racemic):

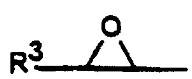

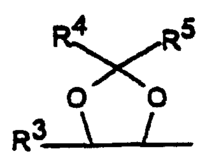

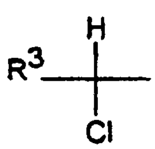

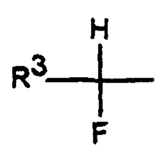

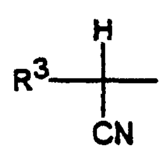

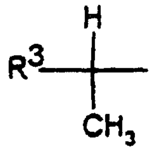

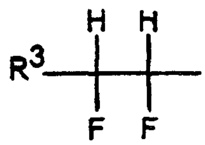

b) hydrogen, where only one of the two radicals $R^1$ and $R^2$ can be H,

with the proviso that $R^1$ must not be bonded to the pyridine ring via -CO-O- or -O-CO-O-;
$R^3$, $R^4$ and $R^5$ are identical or different and are hydrogen or a straight-chain or branched alkyl radical having 1-16 carbon atoms (with or without an asymmetrical carbon atom), where, in addition, one or more non-adjacent, non-terminal $CH_2$ groups may be replaced by -O-, and/or where one or more H atoms of the alkyl radical may be substituted by -F; $R^4$ and $R^5$ may alternatively together be $-(CH_2)_4-$ or $-(CH_2)_5-$ if they are bonded to a dioxolane system.

**2.** A difluorophenylpyrimidylpyridine derivative as claimed in claim 1, wherein the symbols in the formula (I) are defined as follows:

$R^1$ and $R^2$ are identical or different and are

a) an unbranched or branched alkyl chain having 1 to 16 carbon atoms, where
aa) one or more non-adjacent and non-terminal $CH_2$ groups may be replaced by -O-, -CO-O-, -O-CO-, -O-CO-O- or $-Si(CH_3)_2-$, and/or
ab) one or more H atoms may be replaced by F, and/or
ac) the terminal $CH_3$ group may be replaced by one of the following chiral groups (optically active or racemic):

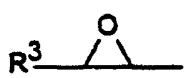

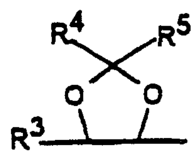

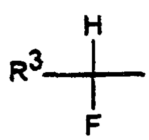

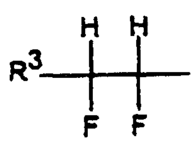

b) hydrogen, where only one of the two radicals R and $R^2$ can be H,

with the proviso that $R^1$ must not be bonded to the pyridine ring via -CO-O- or -O-CO-O-;

$R^3$, $R^4$ and $R^5$ are identical or different and are hydrogen or a straight-chain or branched alkyl radical having 1-13 carbon atoms, where, in addition, one or more non-adjacent, non-terminal $CH_2$ groups may be replaced by -O-, and/or where one or more H atoms of the alkyl radical may be substituted by -F; $R^4$ and $R^5$ may alternatively together be $-(CH_2)_4-$ or $-(CH_2)_5-$ if they are bonded to a dioxolane system.

**3.** A difluorophenylpyrimidylpyridine derivative as claimed in claim 2, wherein the symbols in the formula (I) are defined as follows:

$R^1$ and $R^2$ are identical or different and are

a) an unbranched or branched alkyl chain having 1 to 12 carbon atoms, where
aa) one or more non-adjacent and non-terminal $CH_2$ groups may be replaced by -O-, -CO-O-, -O-CO- or -O-CO-O-, and/or
ab) one or more H atoms may be replaced by F, and/or
ac) the terminal $CH_3$ group may be replaced by one of the following chiral groups (optically active or racemic):

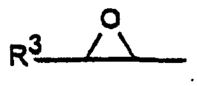

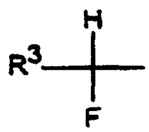

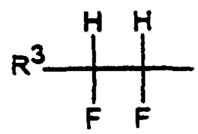

b) hydrogen, where only one of the two radicals R and $R^2$ can be H,

with the proviso that $R^1$ must not be bonded to the pyridine ring via -CO-O- or -O-CO-O-;

$R^3$ is identical or different and is hydrogen or a straight-chain or branched alkyl radical having 1 to 10 carbon atoms, where, in addition, one or two non-adjacent, non-terminal $CH_2$ groups may be replaced by -O-.

**4.** A difluorophenylpyrimidylpyridine derivative as claimed in one or more of the preceding claims, wherein the symbols in the formula (I) are defined as follows:

$R^1$ and $R^2$ are identical or different and are

a) an unbranched or branched alkyl chain having 1 to 12 carbon atoms, where
aa) one or more non-adjacent and non-terminal $CH_2$ groups may be replaced by -O-, -CO-O-, -O-CO- or -O-CO-O-, and/or
ab) the terminal $CH_3$ group may be replaced by one of the following chiral groups (optically active or racemic):

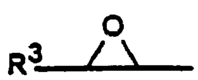

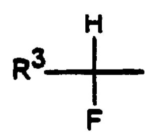

b) hydrogen, where only one of the two radicals $R^1$ and $R^2$ can be H,

with the proviso that $R^1$ must not be bonded to the pyridine ring via -CO-O- or -O-CO-O-;

$R^3$ is identical or different and is hydrogen or a straight-chain or branched alkyl radical having 1 to 10 carbon atoms, where, in addition, one or two non-adjacent, non-terminal $CH_2$ groups may be replaced by -O-.

**5.** The use of a difluorophenylpyrimidylpyridine derivative as claimed in one or more of claims 1 to 4 as a component of liquid-crystalline mixtures.

**6.** A liquid-crystal mixture comprising one or more difluorophenylpyrimidylpyridine derivatives as claimed in one or

more of claims 1 to 4.

**7.** A liquid-crystal mixture as claimed in claim 6, which is ferroelectric.

**8.** A liquid-crystal mixture as claimed in claim 6 and/or 7, which comprises from 0.01 to 80% by weight of one or more difluorophenylpyrimidylpyridine derivatives of the formula (I).

**9.** A ferroelectric switching and/or display device containing a ferroelectric liquid-crystal mixture as claimed in claim 7 and/or 8.

**10.** A ferroelectric switching and/or display device as claimed in claim 9, which is operated in $\tau V_{min}$ mode.

**Revendications**

**1.** Dérivé de difluorophénylpyrimidylpyridine de formule (I)

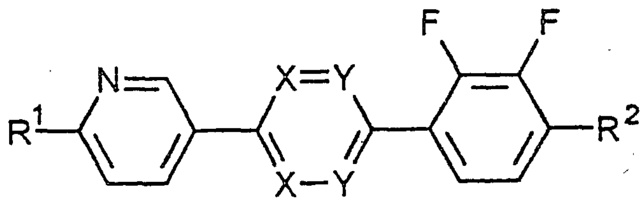

(I)

dans laquelle les symboles ont les significations suivantes :

X est N, et Y est CH, ou X est CH et Y est N ;
$R^1$ et $R^2$ sont identiques ou différents et représentent chacun

a) une chaîne alkyle droite ou ramifiée ayant de 1 à 20 atomes de carbone, où
aa) un ou plusieurs groupes $CH_2$, non voisins et non terminaux, peuvent être remplacés par -O-, -CO-O-, -O-CO-, -O-CO-O-, $-Si(CH_3)_2-$, et/ou
ab) un ou plusieurs atomes d'hydrogène peuvent être remplacés par F, et/ou
ac) le groupe $CH_3$ terminal peut être remplacé par l'un des groupes chiraux (optiquement actifs ou racémiques) suivants :

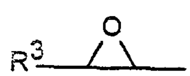

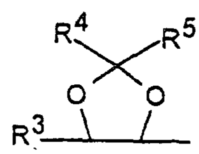

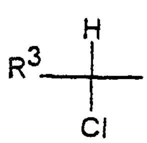

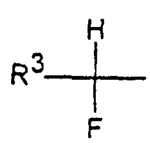

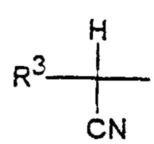

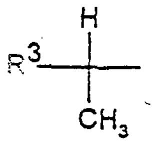
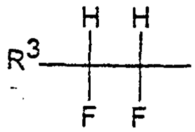

b) un atome d'hydrogène, auquel cas un seul des deux radicaux $R^1$ et $R^2$ peut être H,

à la condition que $R^1$ ne puisse être lié au noyau pyridine par l'intermédiaire de -CO-O- ou de -O-CO-O- ; $R^3$, $R^4$ et $R^5$ sont identiques ou différents et représentent chacun un atome d'hydrogène ou un radical alkyle à chaîne droite ou ramifiée ayant de 1 à 16 atomes de carbone (avec ou sans atome de carbone asymétrique), auquel cas aussi un ou plusieurs groupes $CH_2$ non voisins et non terminaux peuvent être remplacés par -O-, et/ou où un ou plusieurs atomes d'hydrogène du radical alkyle peuvent être substitués par F ; $R^4$ et $R^5$ peuvent aussi former ensemble $-(CH_2)_4$ ou - $(CH_2)_5$, quand ils sont liés à un système dioxolanne.

**2.** Dérivé de difluorophénylpyrimidylpyridine selon la revendication 1, caractérisé en ce que les symboles de la formule (I) ont les significations suivantes :

$R^1$ et $R^2$ sont identiques ou différents et représentent chacun :

a) une chaîne alkyle droite ou ramifiée ayant de 1 à 16 atomes de carbone, où
aa) un ou plusieurs groupes $CH_2$ non voisins et non terminaux peuvent être remplacés par -O-, -CO-O-, -O-CO-, -O-CO-O-, $-Si(CH_3)_2-$, et/ou
ab) un ou plusieurs atomes d'hydrogène peuvent être remplacés par F, et/ou
ac) le groupe $CH_3$ terminal peut être remplacé par l'un des groupes chiraux (optiquement actifs ou racémiques) suivants :

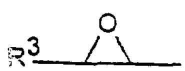
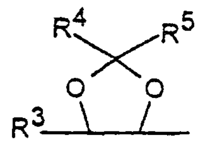

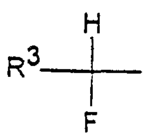
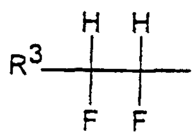

b) un atome d'hydrogène, auquel cas un seul des deux radicaux $R^1$ et $R^2$ peut être H,

à la condition que $R^1$ ne puisse être lié au noyau pyridine par l'intermédiaire de -CO-O- ou de -O-CO-O- ; $R^3$, $R^4$ et $R^5$ sont identiques ou différents et représentent chacun un atome d'hydrogène ou un radical alkyle à chaîne droite ou ramifiée ayant de 1 à 13 atomes de carbone, où aussi un ou plusieurs groupes $CH_2$ non voisins non terminaux peuvent être remplacés par -O-, et/ou où un ou plusieurs atomes d'hydrogène du radical alkyle peuvent être substitués par F ; $R^4$ et $R^5$ peuvent aussi former ensemble $-(CH_2)_4$ ou - $(CH_2)_5$, quand ils sont liés à un système dioxolanne.

**3.** Dérivé de difluorophénylpyrimidylpyridine selon la revendication 2, caractérisé en ce que les symboles de la formule (I) ont les significations suivantes :

$R^1$ et $R^2$ sont identiques ou différents et représentent chacun :

a) une chaîne alkyle droite ou ramifiée ayant de 1 à 12 atomes de carbone, où
aa) un ou plusieurs groupes $CH_2$ non voisins et non terminaux peuvent être remplacés par -O-, -CO-O--O-CO-, -O-CO-O-, et/ou
ab) un ou plusieurs atomes d'hydrogène peuvent être remplacés par F, et/ou
ac) le groupe $CH_3$ terminal peut être remplacé par l'un des groupes chiraux (optiquement actifs ou racémiques) ci-après :

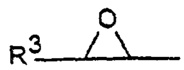

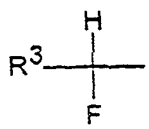

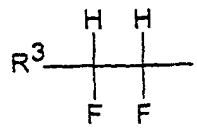

b) un atome d'hydrogène, auquel cas un seul des deux radicaux $R^1$ et $R^2$ peut être un atome d'hydrogène,

à la condition que $R^1$ ne puisse être lié au noyau pyridine par l'intermédiaire de -CO-O- ou de -O-CO-O- ;
$R^3$ est identique ou différent, et représente un atome d'hydrogène ou un radical alkyle à chaîne droite ou ramifiée ayant de 1 à 10 atomes de carbone, auquel cas aussi un ou deux groupes $CH_2$ non voisins et non terminaux peuvent être remplacés par -O-.

**4.** Dérivé de difluorophénylpyrimidylpyridine selon l'une ou plusieurs des revendications précédentes, caractérisé en ce que les symboles de la formule (I) ont les significations suivantes :

$R^1$ et $R^2$ sont identiques ou différents et représentent chacun :

a) une chaîne alkyle droite ou ramifiée ayant de 1 à 12 atomes de carbone, où
aa) un ou plusieurs groupes $CH_2$ non voisins et non terminaux peuvent être remplacés par -O-, -CO-O-, -O-CO-, -O-CO-O-, et/ou
ab) le groupe $CH_3$ terminal peut être remplacé par l'un des groupes chiraux (optiquement actifs ou racémiques) ci-après :

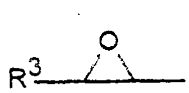

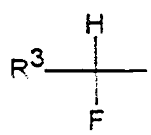

b) un atome d'hydrogène, auquel cas un seul des deux radicaux $R^1$ et $R^2$ peut être un atome d'hydrogène,

à la condition que $R^1$ ne puisse être lié au noyau pyridine par l'intermédiaire de -CO-O- ou de -O-CO-O- ;
$R^3$ est identique ou différent et représente un atome d'hydrogène ou un radical alkyle à chaîne droite ou ramifiée ayant de 1 à 10 atomes de carbone, où aussi un ou deux groupes $CH_2$ non voisins et non terminaux peuvent être remplacés par -O-.

**5.** Utilisation de dérivés de difluorophénylpyrimidylpyridine selon l'une ou plusieurs des revendications 1 à 4, en tant que composants de mélanges de cristaux liquides.

**6.** Mélange de cristaux liquides, contenant un ou plusieurs dérivés de difluorophénylpyrimidylpyridine selon l'une ou plusieurs des revendications 1 à 4.

**7.** Mélange de cristaux liquides selon la revendication 6, caractérisé en ce qu'il est ferroélectrique.

8. Mélange de cristaux liquides selon la revendication 6 et/ou 7, caractérisé en ce qu'il contient de 0,01 à 80 % en poids d'un ou plusieurs dérivés de difluorophénylpyrimidylpyridine de formule (I).

9. Dispositif de commutation et/ou d'affichage ferroélectrique contenant un mélange de cristaux liquides ferroélectrique selon la revendication 7 et/ou 8.

10. Dispositif de commutation et/ou d'affichage ferroélectrique selon la revendication 9, caractérisé en ce qu'il est exploité en mode $_{T}V_{min}$.